(19) Europäisches Patentamt
European Patent Office
Office européen des brevets

(11) EP 0 838 217 B1

(12) **FASCICULE DE BREVET EUROPEEN**

(45) Date de publication et mention de la délivrance du brevet:
**15.01.2003 Bulletin 2003/03**

(51) Int Cl.[7]: **A61K 7/48**, A61K 7/06, A61K 35/74

(21) Numéro de dépôt: **97400161.2**

(22) Date de dépôt: **23.01.1997**

# (54) Composition cosmétique contenant un antagoniste des récepteurs du neuropeptide Y

Neuropeptid Y Rezeptor Antagonisten enthaltende kosmetische Zusammensetzung

Cosmetic composition containing neuropeptide Y receptor antagonist

(84) Etats contractants désignés:
**AT BE CH DE DK ES FI FR GB GR IE IT LI LU MC NL PT SE**
Etats d'extension désignés:
**AL LT LV RO SI**

(30) Priorité: **23.10.1996 FR 9612916**

(43) Date de publication de la demande:
**29.04.1998 Bulletin 1998/18**

(83) **Déclaration conformément à la règle 28(4) CBE (solution de l'expert)**

(60) Demande divisionnaire:
**01100820.8 / 1 132 463**
**01100818.2 / 1 093 803**

(73) Titulaire: **SANOFI-SYNTHELABO**
**75635 Paris Cédex 13 (FR)**

(72) Inventeurs:
- **Blanc-Ferras, Elisabeth**
  **31450 Donneville (FR)**
- **Bono, Françoise**
  **31300 Toulouse (FR)**
- **Breda, Bernard**
  **78380 Bougival (FR)**
- **Courregelongue, Jean**
  **31120 Portet sur Garonne (FR)**
- **Ducasse, Catherine**
  **78800 Houilles (FR)**
- **Mounier, Rémy**
  **78126 Aulnay-sous-Mauldre (FR)**
- **Paul, Raymond**
  **34980 Saint Vely du Fesc (FR)**
- **Pereillo, Jean-Marie**
  **31120 Portet sur Garonne (FR)**
- **Sabadie, Michel**
  **27300 Bernay (FR)**
- **Serradeil-Le Gal, Claudine**
  **31750 Escalquens (FR)**
- **Vilain, Pol**
  **34570 Saussan (FR)**

(74) Mandataire: **Monain, Patrice et al**
**Sanofi-Synthelabo**
**Département Brevets**
**174 avenue de France**
**75013 Paris (FR)**

(56) Documents cités:

| | |
|---|---|
| **EP-A- 0 356 399** | **WO-A-92/00744** |
| **WO-A-96/14307** | **DE-A- 3 545 985** |
| **GB-A- 2 138 023** | **US-A- 5 116 605** |
| **US-A- 5 128 332** | **US-A- 5 194 259** |
| **US-A- 5 536 499** | **US-A- 5 552 148** |

- **CHEMICAL ABSTRACTS, vol. 124, no. 22, 27 mai 1996 Columbus, Ohio, US; abstract no. 298429, N. TANAKA: "TOPICAL PREPARATIONS FOR IMPROVING ROUGH SKIN" XP002070733 & JP 08 026 962 A (KAO CORP.) 30 janvier 1996**
- **CHEMICAL ABSTRACTS, vol. 124, no. 6, 5 février 1996 Columbus, Ohio, US; abstract no. 66228, J. ICHII: "BATH PREPARATIONS CONTAINING .ALPHA.2-ADRENERGIC ANTAGONISTS" XP002070734 & JP 07 258 068 A (KAO CORP.) 9 octobre 1995**
- **VALET et al.: "Neuropeptide Y and Peptide YY Inhibit Lipolysis in Human and Dog Fat Cells through a Pertussis Toxin-sensitive G Protein", J. Clin. Invest., 85, 1, January 1990, 291-295 (XP-001007649)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une composition cosmétique contenant un antagoniste des récepteurs du neuropeptide Y et un composant α2-antagoniste. Le neuropeptide Y ci-après brièvement désigné « NPY » est un neuromédiateur qui intervient dans un certain nombre de processus physiologiques et pour lequel une implication dans la régularisation de la lipolyse a été démontrée (P. Valet et M. J. Clin. Invest. 1990, *85*, 291-295). Des antagonistes des récepteurs NPY, ci-après, désignés. «NPY-antagonistes », ont été décrits comme médicaments, mais leur efficacité dans le traitement d'une maladie quelconque n'a jusqu'à maintenant jamais été prouvée.

US-A-5,536,499 décrit une composition pour application topique comprenant de l'acide phytique qui est un inositol hexaphosphate et un extrait de Gingko biloba. Selon ce document, l'acide phytique sert à atténuer le phénomène de la cellulite et à traiter l'acné.

US-A-5,552,148 décrit une composition cosmétique à base d'inositol phosphate dispersée dans de la vaseline. Une telle composition présente de multiples activités : cicatrisation de la peau, hydratation, embellissement et éclaircissement...

DE 35 45 985 décrit un véhicule à usage oral, notamment pour l'hygiène bucco-dentaire contenant un silicate de zirconium qui, en combinaison avec des composés du fluor, permet la stabilisation du fluor actif. L'addition de myoinositol phosphate est envisagée pour augmenter l'effet anticaries.

WO 92/00 744 décrit l'utilisation de l'inositol monophosphate pour la préparation d'un médicament efficace en tant qu'antagoniste des neuropeptides Y. Aucune utilisation cosmétique n'est divulguée dans ce document.

US 5,128,332 décrit un procédé de traitement des maladies cardiovasculaires utilisant les isomères de l'inositol triphosphate, reconnus pour leur effet NPY-antagoniste.

US 5,194,259 décrit une composition cosmétique amincissante pour application topique contenant une combinaison de Gingko biloba en tant qu'antagoniste α2 et au moins un autre composé α2 bloquant.

Il a été maintenant trouvé que les NPY-antagonistes peuvent être utilisés pour la préparation de compositions cosmétiques.

Plus particulièrement, il a été trouvé que des compositions cosmétiques contenant un composant NPY-antagoniste et un composant α2-antagoniste peuvent etre utilisées comme régulateurs de la lipolyse/ lipogénèse au niveau de la peau sans pour autant interférer avec les fonctions naturelles de celle-ci.

Ainsi, selon un des ses aspects, la présente invention concerne une composition pour le traitement de la cellulite par application topique, contenant un composant NPY-antagoniste et un composant α2-antagoniste en melange avec un excipient cosmétique. Le NPY-antagoniste contenu dans la composition dans laquelle

- $Ar_1$ représente naphtyle, phényle, quinolyle ou isoquinolyle éventuellement substitués par Cl, F, $(C_1\text{-}C_4)$alkyle, $(C_1\text{-}C_4)$alcoxy, hydroxyle ou $(C_1\text{-}C_4)$dialkylamino ;
- $Ar_2$ représente phényle ou thiényle, éventuellement substitués par Cl, F, $(C_1\text{-}C_4)$alkyle, $(C_1\text{-}C_4)$alcoxy ou hydroxyle ;
- $R_1$, $R_2$ et $R'_2$ représentent indépendamment l'un de l'autre l'hydrogène, $(C_1\text{-}C_4)$alkyle ou bien $R_1$ ne représente rien et N est lié à $Ar_2$ et, éventuellement $R_2$ et $R'_2$ forment une double liaison,

ou bien $R_1$ ou $R_2$ est lié à $Ar_2$ et représente $(C_1\text{-}C_3)$ alkylène ;

- $R_3$ et $R_4$ identiques ou différents, représentent l'hydrogène, $(C_1\text{-}C_4)$alkyle ou forment avec l'atome d'azote auquel ils sont liés un hétérocycle saturé en $(C_5\text{-}C_7)$ choisi parmi pyrrolidine, pipéridine et hexahydroazépine ;
- $Z_1$ représente $(C_1\text{-}C_{12})$alkylène interrompu ou substitué éventuellement par $(C_5\text{-}C_7)$cycloalkyle ou phényle ;
- $Q_1$ représente méthyle, amino, $(C_1\text{-}C_4)$alcoxycarbonylamino, $(C_1\text{-}C_4)$alkylamino, di$(C_1\text{-}C_4)$alkylamino, pyrrolidinyle, pipéridino, morpholino, pipérazinyle, $(C_1\text{-}C_4)$alkyl-4-pipérazinyle, amidino, $(C_1\text{-}C_4)$alkylamidino, guanidino, $(C_1\text{-}C_4)$alkylguanidino, pyridyle, imidazolyle, pyrimidinyle, indolyle, hydroxy, $(C_1\text{-}C_4)$alcoxy, $(C_2\text{-}C_8)$alcoxycarbonyle, N-[amino$(C_1\text{-}C_4)$alkyl]-N-[$(C_1\text{-}C_4)$alkyl]amino, carbamoyle ou phényle éventuellement substitué par Cl, F, $(C_1\text{-}C_4)$ alkyle, $(C_1\text{-}C_4)$ alcoxy ou hydroxyle ;
- $Q_2$ représente l'hydrogène ou $(C_1\text{-}C_4)$alkyle ;
- $Q_3$ représente l'hydrogène ou $(C_1\text{-}C_4)$alkyle ou $Q_1$ et $Q_3$ sont liés pour former un hétérocycle et représentent ensemble $(C_2\text{-}C_3)$alkylène tandis que $Z_1$ ne représente rien, sous forme d'énantiomères purs ou de leurs mélanges en proportions quelconques,

ainsi que leurs sels d'addition avec des acides ;
préparables selon EP 614 911.

II - Les analogues du raloxifène, notamment

a) Les composés de formule (II)

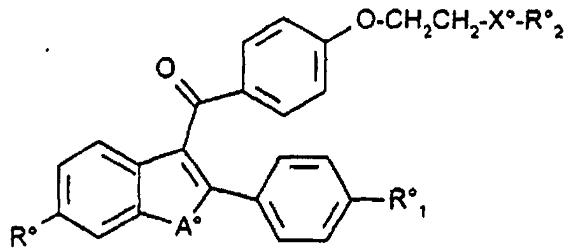

(II)

dans laquelle :

- A° est -O-, $-S(O)_m-$, $-N(R°_6)-$, $-(CH_2)_2-$ ou -CH=CH- ;
- $R°_6$ est l'hydrogène ou un $(C_1-C_6)$alkyle, et m est 0, 1, ou 2;
- X° est une liaison ou un $(C_1-C_4)$alkènylène;
- $R°_2$ est un groupe de formule

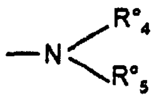

dans laquelle $R°_4$ et $R°_5$ sont indépendamment un $(C_1-C_6)$alkyle ou constituent avec l'atome d'azote auquel ils sont liés, un groupe hétérocyclique choisi parmi hexaméthylèneiminyle, pipérazino, heptaméthylèneiminyle, 4-méthylpipéridinyle, imidazolinyle, pipéridinyle, pyrrolidinyle ou morpholinyle;
- R° est un hydroxy, un halogène, un hydrogène, un $(C_3-C_8)$cycloalkyle, un $(C_2-C_7)$alkanoyloxy, un $(C_1-C_6)$ alcoxy, ou un phényle, ledit phényle étant éventuellement substitué par un, deux ou trois substituants choisis parmi les groupes constitués de $(C_1-C_4)$alkyle, $(C_1-C_4)$alcoxy, nitro, chlore, fluor, trifluorométhyle, $-OSO_2-(C_1-C_{10})$alkyle ou $-O-C(O)-NH-R°_3$;
- $R_1$ est hydroxy, halogène, hydrogène, $(C_3-C_8)$cycloalkyle, $(C_2-C_7)$alcanoyloxy, $(C_1-C_6)$alcoxy, ou phényle, ledit phényle pouvant être éventuellement substitué par un, deux ou trois substituants choisis parmi le groupe constitué de $(C_1-C_4)$alkyle, $(C_1-C_4)$alcoxy, nitro, chlore, fluor, trifluorométhyle, $-OSO_2-(C_1-C_{10})$alkyle ou -O-C(O)-NH-$R°_3$;

dans lequel chaque $R°_3$ représente indépendamment $(C_1-C_6)$alkyle, $(C_3-C_8)$cycloalkyle, phényle non substitué ou substitué dans lequel le substituant est halogène, $(C_1-C_6)$alkyle ou $(C_1-C_6)$alcoxy ; avec la limitation que lorsque X° est une liaison et A° est -S-, alors R° et $R°_1$ ne sont pas tous les deux choisis parmi le groupe constitué de hydroxy, méthoxy et $(C_2-C_7)$alkanoyloxy;
ainsi que ses sels ou solvates, préparables selon WO 96/12489, notamment un composé choisi parmi

· 3-(4-méthoxyphényl)-4-[4-(2-pyrrolidin-1-yléthoxy)benzoyl]-1,2-dihydronaphthalène,
· 3-phényl-4-[4-(2-pyrrolidin-1-yléthoxy)benzoyl]-7-méthoxy-1,2-dihydronaphthaléne,
· 3-(4-méthoxyphényl)-4-[4-[2-(pipéridin-1-yl)éthoxy]benzoyl]-1-,2-dihydronaphthalène,
· 3-(4-hydroxyphényl)-4-[4-[2-(pyrrolidin-1-yl)éthoxy]benzoyl]-1,2-dihydronaphthalène,
· 3-(4-méthoxyphényl)-4-[4-[2-(hexaméthylèneimin-1-yl) éthoxy]benzoyl]-1,2-dihydronaphthalène,
· 3-(4-méthoxyphényl)-4-[4-[2-(pipéridin-1-yl)éthoxy]benzoyl]-1,2-dihydronaphthaléne,
· 3-(4-méthoxyphényl)-4-[4-[2-(pipéridin-1-yl)éthoxylben2oyl]-7-méthoxy-1,2-dihydronaphthalène,
· 3-(4-méthoxyphényl)-4-[4-[2-(N-méthyl-1-pyrrolidinium)éthoxy]benzoyl]-1,2-dihydronaphthaléne,
· 3-(4-méthoxyphényl)-4-[4-(2-diméthylaminoéthoxy)benzoyl]-1,2-dihydronaphthalène,
· 3-(4-méthoxyphényl)-4-(4-diéthylaminoéthoxybenzoyl)-1,2-dihydronaphthaléne,
· 3-(4-méthoxyphényl)-4-(4-diisopropylaminoéthoxybenzoyl)-1,2-dihydronaphthalène,
· 2-(4-hydroxyphényl)-3-[4-[2-(hexaméthylèneimin-1-yl)éthoxy]benzoyl]-6-hydroxybenzofurane,
· 2-(4-hydroxyphényl)-3-[4-[2-(pipérldine-1-yl)éthoxy]benzoyl]-6-hydroxybenzofurane,
· 2-(4-hydroxyphényl)-3-[4-[2-pyrrolidin-1-yl)éthoxy]benzoyl]-6-hydroxybenzofurane,

- 2-(4-hydroxyphényl)-3-[4-[2-(N,N-diéthylamino)éthoxy]benzoyl]-6-hydroxybenzofurane, · 2-(4-hydroxyphényl)-3-[4-[2-(N,N.düsopropylamino)éthoxy)benzoyl]-6-hydroxybenzofurane,
- 2-(4-hydroxyphényl)-3-[4-[2(N,N-diméthylamino)éthoxy]benzoyl]-6-hydroxybenzofurane,
- 1-éthyl-2-(4-méthoxyphényl)-3-[4-[2-(pipéridin-1-yl)éthoxy]benzoyl]-6-hydroxyindole,
- 2-(4-méthoxyphényl)-3-[4-[2-(hexaméthylèneimin-1-yl)éthoxy]benzoyl]-6-méthoxybenzofurane,
- 2-(4-méthoxyphényl)-3-[4-[2-(pipéridin-1-yl)éthoxy]benzoyl]-6-méthoxybenzofurane,
- 2-(4-méthoxyphényl)-3-[4-[2-(pyrrolidin-1-yl)éthoxy]benzoyl]-6-méthoxybenzofurane,
- 2-(4-méthoxyphényl)-3-[4-[2-(N,N-diéthylamino)éthoxy]benzoyl]-6-méthoxybenzofurane,
- 2-(4-méthoxyphényl)-3-[4-[2-(N,N-diisopropylamino)éthoxy]benzoyl]-6-méthoxybenzofurane,
- 2-(4-méthoxyphényl)-3-[4-[2-(N,N-diméthylamino)éthoxy]benzoyl]-6-méthoxybenzofurane,
- 1-éthyl-2-(4-méthoxyphényl)-3-[4-[2(pipéridin-1-yl)éthoxy]benzoyl]-6-méthoxyindole,
- 2-(4-méthoxyphényl)-3-[4-[3-(hexaméthylèneimin-1-yl)propoxy]benzoyl]benzo[b] thiophène,
- 2-(4-méthoxyphényl)-3-[4-[2-(hexaméthylèneimin-1-yl)éthoxy]benzoyl]benzo[b] thiophène,
- 2-(4-méthoxyphényl)-3-[4-[3-(pipéridin-1-yl)propoxy]benzoyl]benzo[b]thiophène,
- 2-(4-méthoxyphényl)-3-[4-[2-(pyrrolidin-1-yl)éthoxy]benzoyl]benzo[b]thiophène,
- 2-(4-méthoxyphényl)-3-(4-(2-(N,N-diéthylamino)éthoxy]benzoyl]benzo[b]thiophène,
- 2-(4-méthoxyphényl)-3-[4-[2-(N,N-diisopropylamino)éthoxyl]benzoyl]benzo[b] thiophène,
- 2-(4-méthoxyphényl)-3-[4-[2-(N,N-diméthylamino)éthoxy]benzoyl]-benzo[b] thiophène,
- 2-(4-chlorophényl)-3-[4-[2-(hexamethylèneimin-1-yl)éthoxy]benzoyl]-6-hydroxybenzo [b]thiophène,
- 2-(4-hydroxyphényl)-3-[4-[2-(pipéridine-1-yl)éthoxy]benzoyl]benzo[b]thiophène,
- 2-(4-hydroxyphényl)-3-[4-[2-(pyrrolidin-1-yl)éthoxy]benzoyl]benzo[b]thiophène,
- 2-(4-hydroxyphényl)-3-[4-[2-(N,N-diéthylamino)éthoxyl]benzoyl]benzo[b]thiophène,
- 2-(4-hydroxyphényl)-3-[4-[2-(N,N-diisopropylamino)éthoxy]benzoyl]benzo[b]thiophène,
- 2-(4-hydroxyphényl)-3-[4-[2-(N,N-diméthylamino)éthoxy]benzoyl]benzo[b]thiophène,
- 2-(4-chlorophényl)-3-[4-[2-(pyrrolidin-1-yl)éthoxy]benzoyl]benzo[b]thiophène-1-oxide,
- 2-(4-chlorophényl)-3-[4-(2(pipéridin-1-yl)éthoxy]benzoyl]benzo[b]thiophène-1-oxide,
- [6-(n-butylsulfonyl)-2-[4-(n-butylsulfonyl)phényl]benzo[b]thién-3-yl][4-[2-(1-pipéridinyl)éthoxy]phénylméthanone,
- [6-(n-pentylsulfonyl)-2-[4-(n-pentylsulfonyl)phényl]benzo[b]thién-3-yl][4-[2-(1-pipéridinyl)éthoxy]phényl]méthanone,
- [6-(n-hexylsulfonyl)-2-[4-(n-hexylsulfonyl)phényl]benzo[b]thién-3-yl][4-[2-(1-pipéridinyl)éthoxy]phényl]méthanone,
- [6-(n-butylsulfonyl)-2-[4-(n-butylsulfonyl)phényl]benzo[b]thién-3-yl][4-[3-(1-pipéridinyl)propyloxy]phényl]méthanone,
- [6-(n-butylsulfonyl)-2-[4-(n-butylsulfonyl]phényl]benzo[b]thién-3-yl]-[4-[2-(1-pyrrolidinyl)éthoxy]phényl]méthanone,
- [6-hydroxy-2-[4-(n-butylsulfonyl)phényl]benzo[b]-thién-3-yl]-[4-[2-(1-pipéridinyl) éthoxy]phényl]méthanone,
- [6-n-butylsulfonyl-2-[4-hydroxyphényl]benzo[b]thién-3-yl]-[4-[2-(1-pipéridinyl) éthoxy]phényl]méthanone,
- [6-[N-(4-chlorophényl)carbamoyl]-2-[4-[N-(4-chlorophényl)carbamoyl]phényl]-benzo[b]thién-3-yl][4-[2-(1-pipéridinyl)éthoxy]phényl]méthanone,
- [6-(N-(n-butyl)carbamoyl]-2-[4-[N-(n-butyl)carbamoyl]phényl]benzo[b]thién-3-yl][4-[2-(1-pipéridinyl)éthoxy] phényl]méthanone,
- [6-(N-méthylcarbamoyl)-2-[4-(N-méthylcarbamoyl)phényl]benzo[b]thién-3-yl][-[2-(1 -pipéridinyl)éthoxy]phényl]méthanone,
- [6-(N-éthylcarbamoyl)-2-[4-(N-éthylcarbamoyl)phényl]benzo[b]thién-3-yl][4-[2-(1 -pipéridinyl)éthoxy]phényl] méthanone,
- [6-(N-isopropylcarbamoyl)-2-[4-[N-isopropylcarbamoyl)phényl]benzo[b]thién-3-yl][4 -[2-(1-pipéridinyl)éthoxy] phényl]méthanone,
- [6-(N-cyclohexylcarbamoyl)-2-[4-(N-cyclohexylcarbamoyl)phényl]benzo[b]thiényl-3 -yl][4-[2-(1-pipéridinyl) éthoxy]phényl]méthanone

et leurs sels et solvates.

(b) Les composés de formule (III)

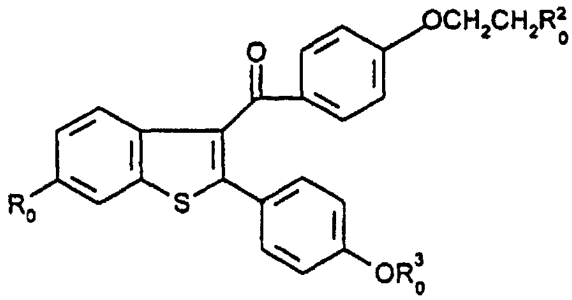

(III)

dans laquelle $R_0$ et $R^3{}_0$, indépendemment, représentent H, $CH_3$, -CO-($C_1$-$C_6$)alkyle, phényle éventuellement substitué et $R^2{}_0$ représente pyrrolidino, pipéridino, hexaméthylènimino ou leurs sels ou solvates pharmaceutiquement acceptables, NPY-antagonistes décrits dans US 5,504,094, notamment le composé de formule (III), dans laquelle $R_0$ et $R^3{}_0$ sont tous les deux l'hydrogène et $R^2{}_0$ est pyrrolidino, ainsi que leurs sels et solvates.

III - Les phénylsulfonylquinoléines de formule (IV) :

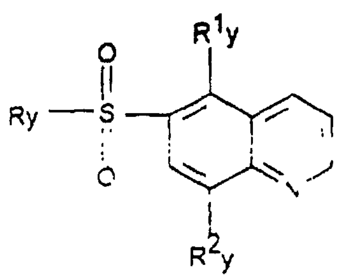

(IV)

dans laquelle Ry est un aryle éventuellement substitué, ou

- $R^1$y est -$NO_2$ -CN, -$CO_2R^3$y dans lequel $R^3$y est H, alkyle ou aryle, -$SO_2R^3$y dans lequel $R^3$y est tel que défini ci-dessus, ou

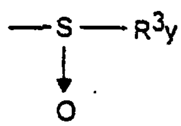

dans lequel $R^3$y est tel que défini ci-dessus, ou

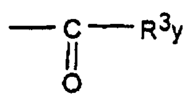

dans lequel $R^3$y est tel que défini ci-dessus,
- $R^2$y est -$NH_2$, -OH ou -SH

et ses sels, décrits dans US 5,552,411, notamment la 8-amino-6-(2-isopropylphénylsulfonyl)5-nitroquinoléine connue sous le code PD-160170 et la 8-amino-6-(4-aminophénylsulfonyl)-5-nitroquinoléine, connue sous le code PD-9262 décrites dans Bioorganic and Médicinal Chemistry Letters, 1996, *6, 15,* 1809-1814.

IV - La bénextramine et ses analogues décrits dans J. Med. Chem. 1993, *36*, 272-279, dans Eur. J. Pharmacology 1994, *37*, 2242-2248 et dans Current Pharmaceutical Design, 1995, I, 295-304, notamment les SC3117, SC3199, CC217 et CC2137.

V - Les dérivés de l'inositol phosphate, notamment :

a) Les isomères spécifiques de l'inositoltriphosphate, notamment le D-myo-inositol-1,2,6-triphosphate, le myo-inositol-1,2,3-triphosphate et le L-myo-inositol-1,3,4-triphosphate, décrits comme NPY-antagonistes dans WO 92/00079 et US 5,128,332.

b) L'inositol monophosphate sous forme acide et de ses sels, notamment de sodium, de potassium, de calcium ou de zinc, décrits comme NPY-antagonistes dans WO 92/00744

VI - Les composés de formule

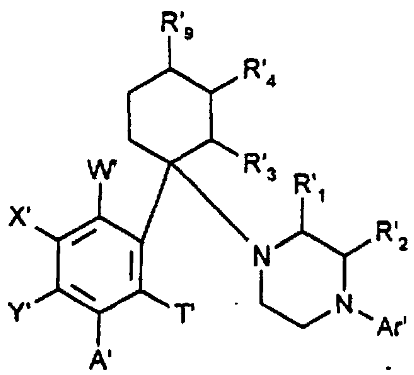

(V)

dans laquelle :

- Ar' est phényle, 2-, 3-, ou 4-pyridyle, 2- ou 3-thiényle, 4- ou 5-pyrimidyle, chacun étant éventuellement mono ou disubstitué par un halogène, un hydroxy, ou une chaîne alkyle inférieure linéaire ou ramifiée ayant de 1 à 6 atomes de carbone ;
- A', W', X', Y', et T' sont identiques ou différents et représentent un hydrogène, un halogène, un hydroxy, une chaîne alcoxy inférieure linéaire ou ramifiée ayant 1 à 6 atomes de carbone ;
- $R'_1$ et $R'_2$ sont identiques ou différents et représentent un hydrogène, une chaîne alkyle inférieure linéaire ou ramifiée ayant 1 à 6 atomes de carbone ;
- $R'_3$ et $R'_4$ sont identiques ou différents et représentent un hydrogène, une chaîne alkyle inférieure linéaire ou ramifiée ayant 1 à 6 atomes de carbone ; et
- $R'_9$ représente un hydrogène, une chaîne alkyle inférieure linéaire ou ramifiée ayant 1 à 6 atomes de carbone, ou un phényle ;

et leurs sels, préparables comme décrit dans WO 96/14307, notamment les

· 1-cyano-1-(4-phénylpipérazin-1-yl)cyclohexane,
· 1-phényl-1-(4-phénylpipérazin-1-yl)cyclohexane,
· 1-(3-méthoxyphényl)-1-(4-phénylpipérazin-1-yl)cyclohexane,
· 1-(3-méthoxyphényl)-1-[4-(2-pyrimidinyl)pipérazin-1-yl]cyclohexane,
· 1-(3-méthoxyphényl)-1-[4-(2-pyridinyl)pipérazin-1-yl]cyclohexane,
· 1-(3-méthoxyphényl)-1-[4-(2-fluorophényl)pipérazin-1-yl]cyclohexane,
· 1-(3-méthoxyphényl)-1-[4-(4-fluorophényl)pipérazin-1-yl]cyclohexane,
· 1-(3-hydroxyphényl)-1-(4-phénylpipérazin-1-yl)cyclohexane,
· 1-(3,5-diméthoxyphényl)-1-(4-phénylpipérazin-1-yl)cyclohexane,
· 1-(3-éthoxyphényl)-1-(4-phénylpipérazin-1-yl)cyclohexane,
· 1-(3-méthoxyphényl)-1-(4-phénylpipérazin-1-yl)-4-phénylcyclohexane,
· 1-(3-*n*-butoxyphényl)-1-(4-phénylplpérazin-1-yl)cyclohexane,
· 1-(3-méthoxyphényl)-1-(4-phénylpipérazin-1-yl)-4-méthylcyclohexane dichlorhydrate isomère cis et isomère trans.

- · 1-(4-méthoxyphényl)-1-(4-phénylpipérazin-1-yl)cyclohexane,
- · 1-(2-méthoxyphényl)-1-(4-phénylpipérazin-1-yl)cyclohexane,
- · 1-(3,4-méthylènedioxyphényl)-1-(4-phénylpipérazin-1-yl)cyclohexane,
- · 1-(3-éthoxyphényl)-1-(4-phénylpipérazin-1-yl)-4-méthylcyclohexane isomère cis et isomère trans,
- · 1-(3-éthoxyphényl)-1-(4-phénylpipérazin-1-yl)-4-éthylcyclohexane isomère cis et isomère trans,
- · 1-(3-isopropoxyphényl)-1-(4-phénylpipérazin-1-yl)-4-méthylcyclohexane isomère cis et isomère trans,
- · 1-(3-méthoxyphényl)-1-(4-phénylpipérazin-1-yl)-3-méthylcyclohexane isomère cis et isomère trans,
- · 1-(3-benzyloxyphényl)-1-(4-phénylpipérazin-1-yl)cyclohexane,
- · 4-(3-éthoxyphényl)-4-(4-phénylpipérazin-1-yl)tétrahydropyrane,
- · 4-(3-éthoxyphényl)-4-(4-phénylpipérazin-1-yl)tétrahydrothiopyrane,
- · 1-(3-méthoxyméthoxyphényl)-1-(4-phénylpipérazin-1-yl)-4-méthylcyclohexane isomère cis et isomère trans,
- · 1-(3-éthoxyméthoxyphényl)-1-(4-phénylpipérazin-1-yl)-4-méthylcyclohexane isomère cis et isomère trans,
- · 1-(3-éthoxyphényl)-1-(4-phénylpipérazin-1-yl)-4-méthoxycyclohexane isomère cis et isomère trans,

VII - Les composés de formule

$$T_a\text{-}Z_a\text{-}NR^1{}_a\text{-}(R^2{}_aCR^3{}_a)\text{-}CO\text{-}Y_a\text{-}(CH_2)_{na}\text{-}R_a \qquad (VI)$$

dans laquelle

- $_{na}$ représente 0, 1, 2, 3, 4, ou 5;
- $R_a$, représente un atome d'hydrogène, un groupe phényle ou naphtyle éventuellement mono- ou disubstitué par un substituant identique ou différent choisi parmi un atome de fluor, de chlore, de brome ou un atome d'iode, un groupe cyano, alkyle, phényle, hydroxy, alcoxy, dialkylaminoalcoxy, hydroxyphényle, phénylalcoxy, alkylcarbonyle, amino, alkylamino, dialkylamino, alkylsulfonylamino, alkylcarbonylamino, alcoxycarbonylamino, alcoxycarbonyloxy, carboxy, alcoxycarbonyle, aminocarbonyle, alkylaminocarbonyle, dialkylaminocarbonyle, alkylcarbonyloxy, alkylsulfonyloxy, hydroxyméthyle, hydroxyéthyle, hydroxypropyle, hydroxybutyle, trifluorométhyle, trifluorométhoxy, trifluorométhylthio, aminoalkyle, alkylaminoalkyle, aminocarbonylaminoalkyle, benzoylamino, alkanoylaminoalkyle, alcoxycarbonylaminoalkyle, benzyloxycarbonylaminoalkyle, aminosulfonyle, alkylaminosulfonyle, dialkylaminosulfonyle, aminosulfonylamino, alkylaminosulfonylamino,
  dialkylaminosulphonylamin, cyanamino, aminocarbonylamino, alkylaminocarbonylamino, dialkylaminocarbonylamino, aminosulfonylaminoalkyle, alkylaminosulfonylaminoalkyle, carboxyalkyle, alcoxycarbonylalkyle, aminocarbonylalkyle, alkylaminocarbonylalkyle, aminosulfonylalkyle, alkylaminosulfonylalkyle, alkylsulfonyle, aminosulfonyloxy, alkylaminosulfonyloxy, dialkylaminosulfonyloxy ou un groupe cyanoguanidino,
  un groupe aminophényle ou aminonaphtyle disubstitué par un atome de chlore ou de brome, les substituants pouvant être identiques ou différents, ou
  un groupe hydroxyphényle ou hydroxynaphthyle disubstitué par un atome de chlore ou de brome ou par des groupes alkyle ou alcoxy, les subtsituants pouvant être identiques ou différents,
  un groupe diphénylméthyle, un groupe aminocarbonylalkyle substitué sur la partie alkyle par un groupe hydroxyphénylalkyle ou par un groupe (2,2-diphényléthyl)aminocarbonylaminophényle,
  un groupe hétéroaryle à 5 chaînons relies par un atome de carbone ou d'azote, ledit hétéroaryle comprenant un groupe imino éventuellement substitué par un groupe $(C_1\text{-}C_6)$alkyle ou un groupe phényle et un atome d'oxygène, de soufre ou d'azote,
  ou un groupe hétéroaryle à 6 chaînons relié par un atome de carbone, ledit hétéroaryle comprenant un ou deux atomes d'azote.
  ainsi que les cycles hétéroaromatiques à 5 ou 6 chaînons définis ci-dessus qui constituent un bicycle hétéroaromatique avec deux atomes de carbone voisins du cycle hétéroaromatique et un groupe 1,4-butadiénylène.
  et de plus, tous les groupes hétéroaryles mono- ou bicycliques mentionnés ci-dessus peuvent être monosubstitués sur le squelette de carbone par un atome de fluor, de chlore ou de brome, par un groupe alkyle, alcoxy, hydroxy, phényle, nitro, cyano, carbonyle, alcoxycarbonyle, aminocarbonyle, alkylaminocarbonyle, dialkylaminocarbonyle, fluorométhyle, difluorométhyle, trifluorométhyle, alkanoyle, aminosulfonyle, alkylaminosulfonyle ou dialkylaminosulfonyle, ou disubstitué par un atome de fluor, de brome ou de chlore, par des groupes méthyle, méthoxy ou hydroxy, les substituants pouvant être identiques ou différents,
  un groupe phényle substitué par un (1,5-dihydro-2,4(3*H*)-dioxo-imidazol-3-yl)alkyle ou par un reste [1,2-dihydro-3,5(4*H*)-dioxo-3*H*-1,2,4-triazol-4-yl]alkyle, dans lequel la partie imidazole ou triazole est substituée par 1 ou 2

groupes phényle,
un groupe cycloalkyle ayant de 4 à 8 atomes de carbone éventuellement substitué par un groupe hydroxy, amino, alkylamino ou dialkylamino, les substituants décrits ci-dessus n'étant pas liés à la position 1 du reste cycloalkyle, lorsque Y représente un atome d'oxygène,
un groupe 1-[[[5,11-dihydro-6(6*H*)-oxo-pyrido[2,3-b][1,4]benzodiazepin-11-yl]carbonyl]méthyl]-4-pipéridinyle ou un groupe 3-hydroxy-1-propin-1-yl, un groupe 2,3-dihydro-1*H*-isoindol-2-yle éventuellement substitué sur l'atome d'azote par un groupe diphénylaminocarbonyle,
ou un groupe hydroxy, lorsque $Y_a$ est un atome d'oxygène ou un groupe -$NR^4{}_a$- et $n_a$ représente un chiffre entre 2 et 5,

- R $_3$ est un atome d'hydrogène, un groupe ($C_1$-$C_{10}$)alkyle linéaire ou ramifié un groupe cycloalkyle ayant de 3 à 7 atomes de carbone, un groupe phényle éventuellement substitué par un groupe hydroxy ou hydroxyalkyle, ou un groupe phénylméthyle éventuellement substitué sur la partie phényle par un groupe hydroxy ou hydroxyalkyle,

- $R^2{}_a$ est un ($C_1$-$C_5$)alkyle non ramifié substitué en postion ω par un groupe amino ou alkylamino protège par un groupe protecteur du groupe amine, par un groupe dialkylmino, N-alkylbenzylamino, aminocarbonyle, aminocarbonylamino, aminoéthylimino, aminoiminométhyle, amino(hydroxyimino)méthyle, amino(alcoxyimino)méthyle, guanidino, hydrazinoiminométhyle, amino(nitroimino)méthyle, [amino(nitroimino)méthyl]amino, amino(cyanimino) méthyle, [amino(cyanimino)méthyl]amino, [(alkylamino)iminométhyl]amino, [(alkylamino)(alkylimino)méthyl]amino, [amino(alkylimino)méthyl]amino, 2-amino-imidazol-1-yle, (5-amino-4*H*-1,2,4-triazol-3-yl)amino, (5-amino-4*H*-1,2,4-triazol-3-yl)méthylamino, (3-amino-1,2,4-oxadiazol-5-yl)amino ou (5-amino-1,2,4-oxadiazol-3-yl)amino ou par un groupe imidazol-4-yle, imidazol-2-yle, 1-méthyl-imidazol-2-yle, imidazol-2-yl-amino, imidazol-2-yl-méthylamino, (4,5-dihydro-1*H*-imidazol-2-yl)amino éventuellement substitué sur le carbone par un ou deux groupes méthyles, ou un groupe phényle ou phénylméthyle éventuellement substitué sur les cycles aromafiques par un groupe cyano, iminométhylamino, cyaniminométhylamino, (méthylamino)méthylidènamino, aminoiminométhyle, amino-(hydroxyimino)méthyle, amino(alcoxyimino)méthyle, hydrazinoiminométhyle, amino (cyanimino)méthyle ou guanidino ou par un groupe imidazol-2-yle, 1-méthyl-imidazol-2-yle, 4,5-dihydro-1*H*-imidazol-2-yle éventuellement substitué sur les atomes de carbone par un ou deux groupes méthyles,
dans lesquels dans les groupes aminoiminométhyles, amino(hydroxyimino)méthyle et guanidino mentionnés dans la définition de $R^2{}_a$ ci-dessus, un ou plusieurs atomes d'hydrogène reliés à l'atome d'azote, indépendants les uns des autres, peuvent être remplacés par des groupes alkyles ou dans lesquels deux atomes d'hydrogène reliés à différents atomes d'azote peuvent remplacés par un pont alkyle ayant de 2 à 4 atomes de carbone et pour lesquels l'atome d'hydrogène d'un groupe HN<, HN= ou $H_2N$ dans le reste $R^2{}_a$ peut être substitué par un groupe alcoxycarbonyle ayant de 2 à 7 atomes de carbone, par un groupe phénylalkoxycarbonyle dans lequel l'alkyle est en ($C_1$-$C_6$), par un groupe phényloxycarbonyle, par un $R^{15}{}_a$-CO-O-($R^{16}{}_a CR^{17}{}_a$)-O-CO ou par un groupe ($R^{18}{}_a$O)PO ($OR^{19}{}_a$) dans lequel :

  - ♦ $R^{15}{}_a$ est un groupe ($C_1$-$C_{15}$)alkyle, un groupe cycloalkyle en ($C_3$-$C_7$), un groupe phényle ou un groupe phénylalkyle dans lequel l'alkyle est en ($C_1$-$C_3$),

  - ♦ $R^{16}{}_a$ et $R^{17}{}_a$, identiques ou différents, représentent un atome d'hydrogène, un groupe ($C_1$-$C_6$)alkyle, ou bien un des restes $R^{16}{}_a$ et $R^{17}{}_a$ représentent un groupe cycloalkyle en ($C_3$-$C_7$) ou un groupe phényle,

  - ♦ $R^{18}{}_a$ et $R^{19}{}_a$, identiques ou différents, représentent un atome d'hydrogène, des groupes ($C_1$-$C_4$)alkyles, des groupes benzyle ou phényles,

- $R^3{}_a$ est un atome d'hydrogène, un groupe ($C_1$-$C_7$)alkyle ou un groupe cycloalkyle en ($C_4$-$C_7$),

- $T_a$ est un atome d'hydrogène, un groupe phényle ou un groupe hétéroaryle à 5 chaînons reliés par un atome de carbone ou d'azote et comprenant un atome d'azote, d'oxygène ou de soufre éventuellement substitués par un groupe alkyle, ou comprenant un atome d'azote éventuellement substitué par un groupe alkyle ainsi qu'un atome de soufre, d'oxygène ou d'azote supplémentaire, ou encore, lorsque $Z_a$ représente une liaison, $T_a$ est un groupe protecteur d'un groupe amino ou les restes ($T^1{}_a T^2{}_a U_a$)-($CH_2$)m- ou $T^3{}_a$O-, dans lesquels

  - ♦ $T^1{}_a$ à $T^3{}_a$, identiques ou différents sont des groupes phényles ou des groupes hétéroaryles à 6 chaînons reliés par des atomes de carbone, qui suivant le cas comprennent un ou deux atomes d'azote,
des groupes hétéroaryles à 5 chaînons reliés par des atomes de carbone ou d'azote, comprenant un atome d'azote éventuellement substitué par un groupe alkyle, de soufre ou d'oxygène, ou comprenant un atome

d'azote
éventuellement substitué par un groupe alkyle ainsi qu'un atome d'azote, de soufre ou d'oxygène supplémentaire,
dans lesquels un pont 1,4-butadiénylène peut être constitué entre deux atomes de carbone voisins des groupes hétéroaryles à 5 ou 6 chaînons mentionnés ci-dessus dans la définition des restes Ta ; les cycles aromatiques ou hétéroaromatiques bicycliques ainsi constitués peuvent être aussi reliés à un atome de carbone du groupe 1,4-butadiénylène et par ailleurs aussi bien les groupes phényles, les groupes hétéroaryles à 5 ou 6 chaînons que les cycles aromatiques ou hétéroaromatiques bicycliques peuvent être mono ou disubstitués sur le squelette carboné par des atomes de fluor, de chlore, de brome ou d'iode ou par des groupes cyano, hydroxy, amino, diméthylamino, diéthylamino, N-éthyl-méthylamino, trifluorométhyle, trifluorométhoxy, trifluorométhylthio, acétylamino, propionylamino, méthanesulfonylamino, méthansulfonyloxy, phényle, phénylméthoxy, 2-phényléthoxy, alkyle, ou alcoxy ou trisubstitués par un groupe amino ou hydroxy et deux atomes de chlore ou de brome ou par un groupe hydroxy et deux groupes alkyles ou alcoxy, les substituants étant identiques ou différents, et les parties alkyles et alcoxy mentionnées ci-dessus comprenant de 1 à 4 atomes de carbone,

des atomes d'hydrogène, des groupes $(C_1-C_{12})$alkyles, des groupes cycloalkyles ayant de 3 à 10 atomes de carbone, des groupes bicycloalkyles ou tricycloalkyles ayant de 6 à 12 atomes de carbone ou

- ♦ $T^1_a$ et $T^2_a$ ensemble représentent un groupe $(C_3-C_7)$alkylène à chaîne linéaire,

- ♦ $U_a$ est le groupe >CH, dans lequel l'atome d'hydrogène peut être remplacé par un groupe alkyle, phényle, hydroxy, alcoxy, alkanoyloxy, alcoxycarbonyle alkanoylamino, dans lequel les alkyles ou les alcoxys mentionnés ci-dessus peuvent comprendre suivant le cas de 1 à 3 atomes de carbone et les alkanoyles mentionnés ci-dessus peuvent comprendre 2 ou 3 atomes de carbone, ou bien le groupe $>CHCH_2$ - ou l'atome d'azote,

- ♦ m représente 0, 1, 2, ou 3

- ♦ ou bien $T_a$ est un groupe $(T^1_aT^2_aU_a)-(CH_2)_{ma}$ dans lequel,

- ♦ $T^1_a$, $T^2_a$, $U_a$ et ma sont tels que définis ci-dessus à la différence que les cycles aromatiques ou hétéroaromatiques mono- ou bicycliques mentionnés ci-dessus pour $T^1_a$ et $T^2_a$ sont liés entre eux par une liaison, par un pont $-CH_2-$, $-C(CH_3)_2-$, $-CH_2CH_2-$, -CH=CH- ou -NHCO-,

- $Y_a$ est un atome d'oxygène ou un groupe $-NR^4_a$, dans lequel $R^4_a$ reprend la définition mentionnée ci-dessus de $R^1_a$, les restes de $R^1_a$ et de $R_{4a}$ pouvant être identiques ou différents, et

- $Z_a$ est un liaison simple définie par un groupe -CO-, $-CH_2-$, -SO-, ou $-SO_2-$,

et, sauf mention contraire les parties alkyles et alcoxy mentionnées ci-dessus peuvent comprendre de 1 à 3 atomes de carbone,
et les tautomères, les diastéréomères, les énantiomères, les mélanges et les sels, décrits dans WO 94/17035.

Parmi ces composés, les

- (R)-$N^2$-(diphénylacétyl)-N-(phénylméthyl)argininamide,
- (R)-$N^2$-(diphénylacétyl)-N-[(4-méthylphényl)méthyl]argininamide,
- (R)-N-[2-(4-hydroxyphényl)éthyl]-$N^2$-[[5-(2-phényléthoxy)-1*H*-indol-2-yl]carbonyl] argininamide,
- N-[(4-aminocarbonylaminophényl)méthyl]-$N^2$-(dipbénylacétyl)argininamide,
- (R)-N-[(4-hydroxyphényl)méthyl]-$N^2$-[[5-(2-phénylethoxy)-1*H*-indol-2-yl]carbonyl] arginamide,
- (R)-$N^2$-(diphénylacétyl)-N-[(4-fluorophértyl)méthyl]argirtinamide,
- (R)-N-[(4-bromophényl)méthyl]-$N^2$-(diphénylacétyl)argininamide,
- (R)-$N^2$-(diphénylacétyl)-N-(2-phényléthyl)argininamide,

les diastéréoisomères optiquement actifs des $N^2$-(α-cyclopentylphénylacétyl)-N-[(4-hydroxyphényl)méthyl]-D-argininamide,

- N-[[4-(diméthylamino)phényl]méthyl]-$N^2$-(diphénylacétyl)argininamide,
- (R)-$N^2$-(diphénylacétyl)-N-[[4-(hydroxyméthyl)phényl]méthyl]argininamide,
- (R)-$N^2$-(diphénylacétyl)-N-[[4-(1-oxoéthyl)phényl]méthyl]argininamide,

- (R)-N-[(4-chlorophényl)méthyl]-$N^2$-(diphénylacétyl)argininamide,
- (R)-$N^2$-(diphénylacétyl)-N-[[4-[(méthylaminocarbonyl)amino]phényl]méthyl] argininamide,
- (R)-N-[(4-amino-3,5-dichlorphényl)méthyl]-$N^2$-(diphénylacétyl)argininamide,
- (R)-N-[(4-amino-3,5-dichlorphényl)méthyl]-$N^2$-(3,3-diphényl-1-oxopropyl) argininamide,
- (R)-N-[(4-amino-3,5-dichlorphényl)méthyl]-$N^2$-(3,4-dichlorobenzoyl)argininamide,
- $N^2$-(diphénylacétyl)-N-[3-[1-[2-[5,11-dihydro-6(6*H*)-oxopyrido[2,3-b][1,4]benzodiazepin-5-yl]-2-oxoéthyl]-4-pipéridinyl]propyl]argininamide,
- $N^2$-(diphénylacétyl)-N-[(4-hydroxy-3-méthoxyphényl)méthyl]argininamide,
- $N^2$-(diphénylacétyl)-N-[(3-hydroxy-4-méthoxyphényl)méthyl]argininamide,
- (R,S)-N-[(4-amino-3,5-dibromphényl)méthyl]-$N^6$-(aminoiminométhyl)-$N^2$-(diphénylacétyl)lysinamide,
- N-[(3,5-diméthyl-4-hydroxyphényl)méthyl]-$N^2$-(diphénylacétyl)argininamide,
- N-[(1*H*-benzimidazol-5-yl)méthyl]-$N^2$-(diphénylacétyl)argininamide,
- $N^2$-(diphénylacétyl)-N-[(4-hydroxy-3-méthylphényl)méthyl]argininamide,
- N-[[4-(aminocarbonyl)phényl]méthyl]-$N^2$-(diphénylacétyl)argininamide,
- (R,S)-$N^6$-(aminoiminométhyl)-$N^2$-(diphénylacétyl)-N-[(4-hydroxyphényl) méthyl]lysinamide,
- (R)-N-[(4-hydroxyphényl)méthyl]-$N^2$-(phénylacétyl)argininamide,
- $N^2$-(diphénylacétyl)-N-[(1*H*-indol-5-yl)méthyl]arginiamide,
- (R)-N-[[4-(aminosulfonyl)phényl]méthyl]-$N^2$-(diphénylacétyl)argininamide,
- (R)-$N^2$-(diphénylacétyl)-N-[[4-méthoxycarbonyl)phényl)méthyl]argininamide,
- (R)-$N^2$-(diphénylacétyl)-N-[(4-pyridinyl)méthyl]argininamide,
- (R)-N-[(4-amino-3,5-dibromphényl)méthyl]-$N^2$-(diphénylacétyl)argininamide,
- (R)-$N^2$-(diphénylacétyl)-N-[(2-thiényl)méthyl]-$N^2$-(2-naphtoyl)argininamide,
- (R)-N-[(4-amino-3,5-dichlorphényl)méthyl]-$N^2$-(2-naphtoyl)argininamide,
- (R,S)-$N^5$-(4,5-dihydro-1*H*-imidazol-2-yl)-$N^2$-(diphénylacétyl)-N-[(4-hydraxyphényl) méthyl]omithinamide,
- (R,S)-$N^2$-(diphénylacétyl)-N-[(4-hydroxyphényl)méthyl]-$N^5$-(1*H*-imidazol-2-yl) ornithinamide,
- (R)-N-[[3-[(1,2-dihydro-3,5(4*H*)-dioxo-1,2-diphényl-3*H*-1,2,4-triazol-4-yl)méthyl] phényl]-$N^2$-(diphénylacétyl)argininamide,
- $N^2$-(diphénylacétyl)-N-[(3-hydroxyphényl)méthyl]argininamide,
- (R)-N-[[3-[(4,5-dihydro-2,4(3*H*)-dioxo-5,5-diphényl-1*H*-imidazol-3-yl)méthyl]phényl] methyl]-$N^2$-(diphénylacétyl) argininamide,
- (R)-$N^2$-(diphénylacétyl)-N-[(4-hydroxyphényl)méthyl]argininamide, connu sous son code BIBP3226,
- $N^2$-(diphénylacétyl)-N-[2-(4-hydroxyphényl)èthyl]argininamide,
- $N^2$-(diphénylacétyl)-[(4'-hydroxy-[1,1'-biphényl]-4-yl)méthyl]argininamide,
- N-[[4-[(1,2-dihydro-3,5(4*H*)-dioxo-1,2-diphényl-3*H*-1,2,4-triazol-4-yl)méthyl]phényl] méthyl]-$N^2$-(diphénylacétyl) argininamide,
- $N^2$-(diphénylacétyl)-N-[(4-méthoxyphényl)méthyl]argininamide,
- $N^2$-(diphénylacétyl)-N-[2-(4-méthoxyphényl)éthyl]argininamide,
- $N^2$-(diphénylacétyl)-N-[2-(3-méthoxyphenyl)éthyl]argininamide,
- $N^2$-(diphénylacétyl)-N-[(3-méthoxylphényl)méthyl]argininamide
- (R,S)-3-[4-(aminoiminométhyl)phényl]-$N^2$-(diphénylacétyl)-N-[(4-hydroxyphényl) méthyl]alaninamide,
- (R,S)-3-[3-(aminoiminométhyl)phényl]-$N^2$-(diphénylacétyl)-N-[(4-méthoxyphényl) méthyl]alaninamide,
- (R,S)-3-[3-(aminoiminométhyl)phényl]-$N^2$-(diphénylacétyl)-N-[(4-hydroxyphényl) méthyl]alaninamide,
- (R)-$N^2$-[bis-(4-bromophényl)acétyl]-N-[(4-hydroxyphényl)méthyl]argininamide,
- (R)-$N^2$-(diphénylacétyl)-N-[(4-éthoxyphényl)méthyl]argininamide,
- (R)-$N^2$-(diphénylacétyl)-N-[(4-hydroxyphényl)méthyl]-N-méthylargininamide,
- (R,S)-N-[(4-amino-3,5-dibromphényl)méthyl]-3-(3-(aminoiminométhyl)phényl]-$N^2$ -(diphénylacétyl)alaninaminde,
- (R)-N-[(4-hydroxyphényl)méthyl]-$N^2$-(2-naphtoyl)argininamide,
- (R)-N-[(4-hydroxyphényl)méthyl]-$N^2$-(2-naphtoyl)argininamide,
- (R)-$N^2$-(2,2-diphényl-2-hydroxyacétyl)-N-[(4-hydroxyphényl)méthyl]argininamide,
- (R,S)-$N^2$-(diphénylacetyl)-$N^5$-(1*H*-imidazol-2-yl)-N-[(4-méthoxyphényl)méthyl] ornithinamide,
- (R,S)-3-[3-(aminoiminométhyl)phényl]-$N^2$-[[(diphénylméthyl)amino]carbonyl]-N-[(4-hydroxyphényl)méthyl]alaninamide,
- (R,S)-$N^2$-(diphénylacetyl)-$N^5$-(1*H*-imidazol-2-yl)-N-(phénylméthyl)-ornithinamine,
- (R,S)-N-[(4-amino-3,5-dichlorphényl)méthyl]-$N^2$-(diphénylacetyl)-$N^5$-(1*H*-imidazol-2 -yl)-ornithinamide,
- (R,S)-N-[(4-hydroxyphényl)méthyl]-$N^5$-(1*H*-imidazol-2-yl)-$N^2$-(2-naphthoyl) -ornithinamide,
- (R,S)-$N^2$-[[(diphénylméthyl)amino]carbonyl]-N-[(4-hydroxyphényl)méthyl]-$N^5$-(1*H* -imidazol-2-yl)ornithinamide,
- (R,S)-N-[(4-hydroxyphényl)méthyl]-$N^5$-(1*H*-imidazol-2-yl)-$N^2$-[(2-naphthyl)acétyl] omithinamide,
- (R,S)-N-[(4-hydroxyphényl)méthyl]-$N^5$-(1*H*-imidazol-2-yl)-$N^2$-[[(2-naphthyl)amino] carbonyl]ornithinamide,

- (R,S)-N-[(4-hydroxyphényl)méthyl]-$N^5$-(1*H*-imidazol-2-yl)-$N^2$-[(1,2,3,4-tétrahydroquinoline-3-yl)carbonyl]ornithinamide,
- (R,S)-N-[(4-hydroxyphényl)méthyl]$N^5$-(1*H*-imidazol-2-yl)-$N^2$-[(1,2,3,4-tétrahydroquinoline-2-yl)carbonyl]omithinamide,
- (R)-N-[(4-aminosulfonylaminophényl)méthyl]-$N^2$-(diphénylacétyl)argininamide,
- (R)-N-[(4-aminophényl)méthyl]-$N^2$-(diphénylacétyl)argininamide,
- (R)-N-[(6-quinolinyl)méthyl]-$N^2$-(diphénylacétyl)argininamide,
- (R)-$N^2$-[(3,4-dichlorophényl)acétyl]-N-[(4-hydroxyphényl)méthyl]argininamide,
- (R)-$N^2$-(diphénylacétyl)-N-[[4-(2-hydroxyéthyl)phényl]méthyl]argininamide,
- (R,S)-$N^5$-(3-amino-1,2,4-oxadiazol-5-yl)-$N^2$-(diphénylacétyl)-N-[(4-hydroxyphényl) méthyl]ornithinamide,
- (R)-$N^2$-[(9-fluorényl)carbonyl]-N-[(4-hydroxyphényl)méthyl]argininamide,
- (R,S)-6-(aminoiminométhyl)-$N^2$-(diphénylacétyl)-N-[(4-hydroxyphényl)méthyl] norleucinamide,
- (R,S)-3-[4-(4,5-dihydro-1*H*-imidazol-2-yl)phényl]-$N^2$-(diphénylacétyl)-N-[(4-hydroxyphényl)méthyllalaninamide,
- (R,S)-3-[3-(aminoiminométhyl)phényl]-$N^2$-(diphénylacétyl)-N-[(4-éthoxycarbonyl oxyphényl)méthyl]alaninamide,
- (R,S)-N-[2-(1,2-dihydro-1,2-diphényl-3,5(4*H*)-dioxo-1,2,4-triazol-4-yl)éthyl]-$N^2$ -(diphénylacétyl)argininamide,
- (R,S)-$N^2$-(diphénylacétyl)-N-[(4-hydroxyphényl)méthyl]-2-méthylargininamide,
- (R,S)-$N^2$-(diphénylacétyl)-N-[[4-(3-hydroxypropyl)phényl]méthyl]argininamide,
- (R)-N-[[4-[(4,5-dihydro-5,5-diméthyl-2,4(3*H*)-dioxo-1*H*-imidazol-3-yl)méthyl]phényl] méthyl]-$N^2$-(diphénylacétyl) argininamide,

sont avantageux, le BIBP 3226 étant préféré.

VIII - Les composés de formule

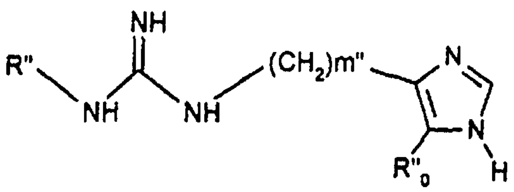

(VII)

dans laquelle R" est défini par la formule :

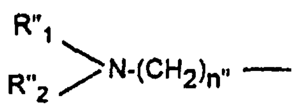

dans laquelle $R''_1$ représente un groupe phényle non substitué ou substitué par un ou deux atomes halogène, un groupe ($C_1$-$C_3$)alkyle ou un cycle pyridinyle non substitué
ou substitué par un ou deux atomes halogène ,un groupe ($C_1$-$C_3$)alkyle ou un groupe ($C_1$-$C_3$)alcoxy,

- $R''_2$ représente un atome d'hydrogène, un groupe ($C_1$-$C_3$)alkyle, un groupe phényle éventuellement substitué par un ou deux atomes d'halogène ou par un groupe ($C_1$-$C_3$)alkyle ou par un groupe ($C_1$-$C_3$)alcoxy, un groupe benzyle ou hétéroaryle non substitué ou substitué par un un ou deux atomes d'halogène ou un groupe ($C_1$-$C_3$)alkyle ou ($C_1$-$C_3$)alcoxy,
- n" représente 1, 2, 3 ou 4

ou dans laquelle R" est défini par la formule :

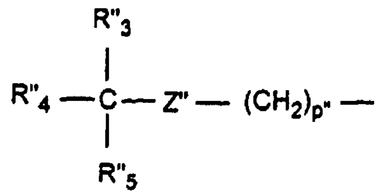

dans laquelle R"$_3$ représente un cycle pyridinyle non substitué ou substitué par un ou deux atomes d'halogène, par un groupe ($C_1$-$C_3$)alkyle ou ($C_1$-$C_3$)alcoxy, R"$_4$ représente un atome d'hydrogène ou un groupe phényle éventuellement substitué par un ou deux atomes d'halogène ou par un groupe ($C_1$-$C_3$)alkyle ou par un groupe ($C_1$-$C_3$)alcoxy, R"$_5$ représente un atome d'hydrogène ou un groupe méthyle ou hydroxy et Z" représente une liaison simple, un atome d'oxygène, ou un atome de soufre et p" représente 1, 2 ou 3 m » représente 1,2 ou 3 et R"$_0$ représente un atome d'hydrogène ou un groupe méthyle, ainsi que leurs sels pharmaceutiquement acceptables, dont l'effet NPY antagoniste est décrit dans EP 448 765.

Parmi ces composés, ceux de formule (VII) dans laquelle m" est 3, R"$_0$ est l'hydrogène et R" est 2-(3-pyridylméthylthio)éthyle, 3,3-diphénylpropyle, 2-(2-pyridylamino)éthyle, 2-(5-bromo-3-méthyl-2-pyridylamino)éthyle, 2-(diphénylméthoxy)éthyle, 3-(3,5-difluorophényl)-3-(2-pyridyl)propyle, 2-[N-(5-bromo-3-méthyl-2-pyridyl)-N-benzylamino] éthyle ou 2-(5-bromo-3-méthyl-2-pyridyl)éthyle et leurs sels sont particulièrement avantageux, le trichlorhydrate de 1-[3-(3,5-difluorophényl)-3-(2-pyridyl)]propyl-3-[4-(1*H*)imidazolyl]guanidine(He 90481) étant préféré.

IX - Les dihydropyridines de formule :

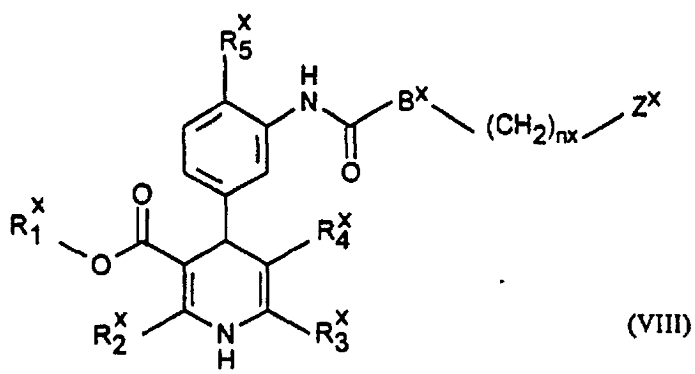

(VIII)

dans laquelle

- $R_1^x$ est un alkyle inférieur ;
- $R_2^x$ et $R_3^x$ sont indépendamment choisis parmi un cyano et un alkyle inférieur ;
- $R_4^x$ est choisis parmi -$CO_2R_1^x$, cyano et

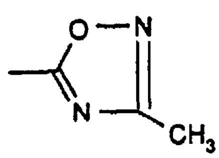

- $R_5^x$ est choisi parmi un hydrogène, un halogène, un hydroxyle, un alkyle inférieur, un alkényloxy inférieur et un alcoxy inférieur ;
- $B^x$ est -NH- ou une liaison covalente ;
- nx représente un nombre entier de 2 à 5 et
- $Z^x$ est choisi parmi le groupe constitué par :

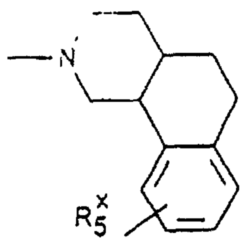 , 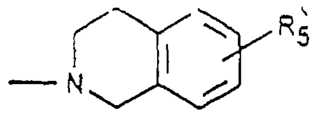 ,

et

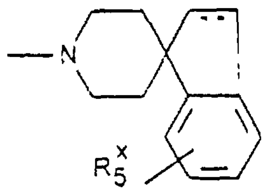

les lignes pleines ou en pointillés représentant une liaison simple ou double, ainsi que leurs sels d'addition ou solvates décrits dans US 5,554,621.

B D'autres NPY-antagonistes avantageux dans les compositions cosmétiques de la présente invention sont les peptides choisis parmi ceux des groupes IX à XII ci-après.

X - Les composés de formule

$$\text{Tyr-Pro-Ser-Lys-Pro-Asp-Cys-NH-}(CH_2)_s\text{COXxx}_2$$

$$\text{-Arg-Cys-Tyr-Xxx}_3\text{-Ala-Leu-Arg-His-Tyr-Xxx}_4$$

$$\text{-Asn-Leu-Xxx}_5\text{-Thr-Arg-Ile-Arg-Tyr-Tc} \qquad \text{(IX)}$$

dans laquelle

- $Xxx_2$ et $Xxx_3$ sont Ser ou Ala, $Xxx_4$ et $Xxx_5$ sont Leu, Ile, Met, Nle (Norleucine) ou Val
- Tc est OH, $O(C_1\text{-}C_4)$alkyle, $NH_2$ ou $NH(C_1\text{-}C_4)$alkyle,
- est un nombre entier de 1 à 11, et leurs sels, décrits dans EP 355794.

Parmi ces composés, celui de formule (IX), dans laquelle s est 7, $Xxx_2$ est Ala, $Xxx_3$ est Ser, $Xxx_4$ et $Xxx_5$ sont tous les deux Ile et Tc est $NH_2$, et ses sels, sont avantageux.

XI - Les composés de formule

$$Z_b\text{-}A_{k1}\text{-}B_{k2}\text{-}BP_{k3}\text{-}D_{k4}\text{-}E_{k5}\text{-}F_{k6}\text{-}G_{k7}\text{-DP-Thr-BP'-Glu}$$

$$\text{-BP''-K-}NH_2 \qquad \text{(X)}$$

dans laquelle $k_1$, $k_2$, $k_3$, $k_4$, $k_5$, $k_6$ et $k_7$ représentent zéro ou 1, leur somme étant au moins 1, A est Phe ou Tyr ; B est Pro, Ser, Ala, Gly, Abu, Cys, Pro-Ser, Cys-Ser, Ala-Ser, Gly-Ser, Abu-Ser ou Pro-Ala, Abu étant l'acide 2-aminobutyrique ; BP, BP' et BP" sont des acides aminés avec une chaîne latérale basique ; D est Pro ou Cys ; E est $NH\text{-}(CH_2)_t\text{-}CO$, t étant 0-10 ; F est Cys, Abu Gly ou Ala; G est Ile-Asn, Leu-Asn, Val-Asn ou Asn; DP est le résidu d'un dipeptide formé par des acides aminés lipophiles ; K est Phe ou Tyr ; $Z_b$ est l'hydrogène, un groupe amino-protecteur

ou un groupe benzyle, $(C_2-C_{10})$acyle ou $(C_1-C_5)$alkyle et dans laquelle deux résidus Cys peuvent être liés par un pont disulfure, ainsi que leurs sels, décrits dans DE 3 939 801.
Parmi ces composés, ceux de formule (X) dans laquelle BP est Lys ou Arg ; E est $NH-(CH_2)t-CO$, avec t = 1-7 ; F est Cys ou Abu ; G est Ile-Asn ou Asn ; DP est Leu-Ile, BP' et BP", identiques, sont Arg ; Zb est hydrogène, acétyle ou un groupe

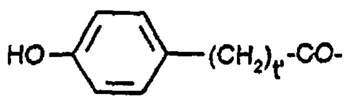

où t' est 0, 1 ou 2, et leurs sels sont avantageux,
le composé de formule

H-Tyr-Abu-Ser-Lys-Aoc-Abu-Ile-Asn-Leu-Ile

$Thr-Arg-Gln-Arg-Tyr-NH_2$ (X')

où Aoc représente l'acide 8-aminooctanoïque étant particulièrement préféré.
XII - Les composés cycliques ou linéaires, de formule

$$(Xxx_a-Xxx_b-Xxx_c-Xxx_d-Xxx_e-Xxx_f-Xxx_g-Xxx_h-Xxx_i-Xxx_j)_n \quad (XI)$$

dans laquelle $Xxx_a$, $Xxx_b$, $Xxx_c$ et $Xxx_l$ peuvent ne pas exister, ou bien $Xxx_a$ est $P_1$ ou Cys ; $Xxx_b$ est Cys, $P_1$ ou un ou deux acides aminés ; Xxxc est $P_1$, Cys, Ser, Thr, Ala, ou Gly ; $Xxx_d$ est $R_1$, Cys, Ser, Thr, Ala ou Gly ; $Xxx_e$ est Leu, Ile, Val ou Nle ; $Xxx_g$ est Arg, Lys ou His ; $Hxx_g$ est Arg, Lys, His, Val, Leu, Ile ou Nle ; $Xxx_h$ est Tyr, Phe, Trp, His, Lys ou Arg ; Xxx, représente un groupe $NH_2$ ou un groupe ester ou bien un ou deux acides aminés ; $Xxx_j$ est Cys ou $P_2$ ; $P_1$ est l'hydrogène ou un groupe P-CO où P est l'hydrogène, un radical glycosyle, nucléosyle ou lipolyle ou un groupe $(C_1-C_{20})$ alkyle, contenant éventuellement des doubles liaisons et des substituants choisis parmi les halogènes et le groupe $NO_2$, $NH_2$, OH, sulfo, phospho, carboxy et alkyle ; $P_2$ est un groupe $NP_{12}P_{13}$ ou $OP_{14}$ dans lequel $P_{12}$ et $P_{13}$ représentent l'hydrogène ou un groupe alkyle, cycloalkyle, N-glycosyle, N-lipolyle, aralkyle ou aryle, éventuellement substitué par un halogène ou par un groupe $NO_2$, $NH_2$, OH, sulfo, phospho, carboxy ou alkyle ; et $P_{14}$ représente l'hydrogène ou un groupe alkyle, cycloalkyle, aralkyle, -O-glycosyle, -O-lipolyle, aralkyle ou aryle, éventuellement substitué par un halogène ou par un groupe $NO_2$, $NH_2$, OH, sulfo, phospho, carboxy ou alkyle, avec la limitation que

- lorsque $Xxx_b$ n'existe pas ou représente Cys ou $P_1$, alors $Xxx_a$ est inexistant,
- lorsque $Xxx_d$ est Cys ou $P_1$, alors $Xxx_c$ est inexistant,
- lorsque $Xxx_i$ est $NH_2$ ou un ester, alors Xxxj est inexistant.

Ces composés sont décrits dans WO 93/12139.
Parmi ceux-ci le peptide de formule :

$Ser-Ala-Leu-Arg-His-Tyr-NH_2$ (XI')

connu sous le numéro de code BRC 672, est particulièrement avantageux.
XIII - Les composés de formule

H-Tyr-Pro-Ser-Lys-Pro-Asp-Asn-Pro-Gly-Glu-

$Asp-Ala-Pro-Ala-Glu-Asp-Xxx_6-Ala-Arg-Tyr-Tyr-$

Ser-Ala-Leu-Arg-His-Tyr-Ile-Asn-Leu-

Ile-Thr-Arg-$Xxx_7$-$Xxx_8$-L1-$(CH_2)_2$-$P_3$ (XII)

dans laquelle z est 0,1 ou 2, $Xxx_6$ est Met ou Leu ; $Xxx_7$ est Glu, (R)-Glu, Pro ou (R)-Pro ; $Xxx_8$ est Arg, (R)-Arg ou (R, S)-Arg ; $L_1$ est O ou NP', P' étant H ou alkyle ; et $P_3$ est

- un groupe phényle ou naphtyle non substitué ou substitué par un ou deux F, Cl, Br, alkyle, phényle, OH, (phényl) alcoxy, alkylcarbonyle, $NH_2$ (éventuellement substitué par un ou deux alkyles), alkylsufonyle, alkyl-ou un alcoxycarbonylamino, COOH, alcoxycarbonyle, NH2CO (éventuellement substitué par un ou deux alkyles), alkylcarbonyloxy, alkylsulfonyloxy, $CH_2OH$, 1- ou 2-hydroxyéthyle, (alkyl)aminosulfonyle, cyanamino, aminocarbonylamino (éventuellement substitué par un ou deux alkyles) ou $NH_2C(=N.CN)NH$ ;
- un hétérocycle aromatique à 5 membres contenant l'oxygène, le soufre ou l'azote, un groupe imino ou un groupe imino contenant l'oxygène, le soufre ou l'azote ;
- un hétérocyle aromatique à 6 membres avec un ou deux atomes d'azote ; lesdits hétérocycles pouvant être C-substitués par ($C_1$-$C_6$)alkyle ou par phénylalkyle et éventuellement condensés au benzène et éventuellement également substitués soit par un F, Cl, Br, ($C_1$-$C_6$)alkyle, alcoxy, OH, phényle, $NO_2$, $NH_2$ (éventuellement substitué par un ou deux alkyles ou par un alcanoyle), CN, COOH, alcoxycarbonyle, $NH_2CO$ (éventuellement substitué par un ou deux alkyles), $CH_2F$, $CHF_2$, $CF_3$, alcanoyle ou $NH_2SO_2$ (éventuellement substitué par un ou deux alkyles), soit par deux F, Cl, Br, méthyle, méthoxy ou OH ;
- un groupe phényle substitué par un groupement [1,5-dihydro-2,4-(3*H*)-dioxoimidazol-3-yl]alkyle ou par un groupement [dihydro-3,5-(4*H*)-dioxo-3*H*-1,2,4-triazole-4-yl]alkyle dont les groupes imidazole et triazole peuvent être substitués par un ou deux phényles ; les goupes alkyle et alcoxy contenant de 1 à 3 atomes de carbone,

et leurs sels.

Ces composés sont illustrés dans DE 4 311 756.

XIV - Les NPY-antagonistes préparables selon WO 94/00486, JP 06-116284, JP 07-267988, DE 3 811 193, US 5,328,899 et WO 95/00161, tels que le composé (XIII)

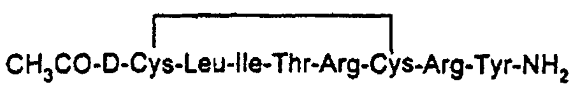

(XIII)

décrit dans JP 06-116 284 et le composé (XIV)

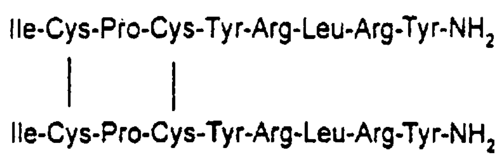

(XIV)

décrit dans WO 94/00486.

C Des produits NPY-antagonistes, notamment des extraits à activité NPY-antagoniste, susceptibles d'être obtenus par extraction de cellules ou de tissus d'origine animale ou végétale ou par fermentation de micro-organismes, notamment de bactéries et de champignons, par exemple des levures sont les composants NPY-antagonistes avantageux dans les compositions cosmétiques de la présente invention.

Des produits de cette classe, particulièrement avantageux, et même préférés sont les produits des groupes XV. XVI et XVII illustrés ci-après.

XV - Les extraits à activité NPY-antagoniste obtenus par extraction d'une sponge, *Orina sp. Gray* (ou *Gellius sp.*), notamment les composés de l'indole décrits dans J. Nat. Products, 1994, 57, 1294-1299 et ibid.1995, *58*, 8, 1254-1260, ou leurs mélanges, plus particulièrement la gelliusine A, la gelliusine B, la 2-[5-hydroxy-3-(2-aminoéthyl)] indol-2-yl-6-bromo-3-indoleéthanamine, la 2-[6-bromo-3-(2-aminoéthyl)] indol-2-yl-6-bromo-3-indoleéthanamine, leurs sels et leurs mélanges.

XVI - Les extraits de fermentations de souches d'*Aspergillus*, par exemple d'*Aspergillus niger à* activité NPY-antagoniste, notamment le composé connu sous le nom de code BMS-192548, préparable par extraction du milieu de culture *d'Aspergillus niger* WB 2346, comme décrit dans J. Antibiotics, 1995, *48, 10,* 1055-1059, ayant la formule (XV)

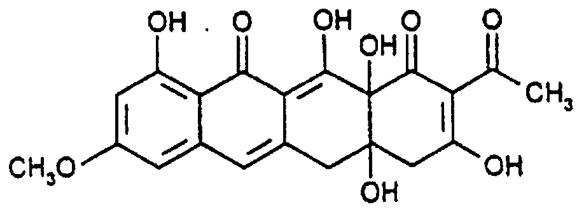

(XV)

Un autre produit à activité NPY-antagoniste utilisable est l'extrait obtenu par fermentation d'une nouvelle souche d'actinomycètes présentant une activité particulièrement intéressante comme antagonistes des récepteurs NPY.

A partir des moûts de fermentation de cette souche, par filtration du surnageant, éventuellement suivie par des étapes de concentration, de purification et/ou de lyophilisation, on obtient des fractions à activité antagoniste des récepteurs NPY qui sont dépourvues de génotoxicité et ont une stabilité suffisante pour permettre de les formuler dans les compositions cosmétiques de la présente invention.

Le surnageant du moût.de fermentation peut aussi être utilisé tel quel.

Ce nouvel extrait constitue un aspect ultérieur et particulièrement avantageux de la présente invention.

L'organisme producteur des extraits à activité antagoniste des récepteurs NPY selon cet aspect particulier de la présente invention, est une souche d'actinomycètes qui a été isolée à partir d'un échantillon de terre de prairie prélevée en Haute Garonne (France), auquel on a attribué le numéro interne SEBR 2794. Un échantillon de ce micro-organisme a été déposé le 13 juillet 1993 auprès de la C.N.C.M. de l'institut Pasteur où il a été enregistré sous la référence I 1332.

Les caractéristiques biochimiques de ce micro-organisme ont été déterminées sur galeries API 50 CH (spécifiques des sucres), API 50 AO (spécifiques des acides organiques) et API 50 AA (spécifiques des acides aminés) (commercialisés par BioMérieux pour API 50CH et par le laboratoire de recherche API, pour les autres).

On a ainsi déterminé que ce micro-organisme appartient à la famille des *Streptomycetaceae,* genre *Streptomyces*.

Il s'agit d'un micro-organisme polymorphe, filamenteux. Il se développe bien à 28°C sur un milieu de culture $ISP_2$ (agar yeast malt extract), la coloration de son mycelium végétatif étant de couleur gris-rosé.

Cet organisme, présentant des caractères qui n'ont pas permis de l'identifier à une espèce déjà décrite, doit être considéré comme une espèce nouvelle et il a été désigné *Streptomyces sp* SEBR 2794.

L'isolement de cette nouvelle souche a été effectué en suivant la méthode usuelle qui consiste à mettre une petite quantité de terre en suspension dans de l'eau distillée, à diluer la suspension à différentes concentrations et à étaler un petit volume de chaque dilution sur la surface d'une boîte de Petri contenant un milieu nutritif gélosé. Après une incubation de quelques jours à 28°C, qui permet aux micro-organismes de se développer, les différentes colonies sont séparément prélevées et repiquées sur les géloses nutritives afin d'en obtenir des cultures plus abondantes.

Après cultures sur milieu nutritif gélosé et plusieurs repiquages successifs qui permettent d'obtenir une culture abondante et pure de la souche d'intérêt, on fabrique un lot 0 de conservation de la souche mère puis des lots de semence primaire et secondaire.

Pour cela, on prépare une suspension de spores à partir d'une culture sur milieu nutritif gélosé en boîte de Pétri et d'un milieu de reprise ; ce milieu contient un cryoprotectant permettant d'assurer une bonne viabilité des spores lors de la conservation par congélation.

La suspension de spores obtenue est répartie dans des cryotubes qui seront conservés à -80°C,: ces tubes constituent le lot 0.

En suivant le même protocole, mais à partir d'un tube du lot 0, on prépare un lot de semence primaire.

Puis toujours selon le même protocole on prépare un lot de semence secondaire à partir d'un cryotube du lot de semence primaire.

La fabrication des lots de semences 0,1 et 2 assure une accessibilité durable de la souche et donc de l'activité recherchée.

Le procédé de préparation des extraits à activité antagoniste des récepteurs du NPY consiste essentiellement à cultiver la nouvelle souche SEBR 2794, ou ses mutants producteurs, sur un milieu et dans des conditions de culture appropriées et à extraire ensuite du moût de fermentation la fraction active produite au cours de la culture ; fraction active qui se trouve dans le sumageant.

La culture de *Streptomyces sp* SEBR 2794 peut être effectuée par toute méthode de culture aérobie. On utilise à cette fin, les différents types d'appareils qui sont d'un usage courant dans l'industrie des fermentations. On peut, en particulier, adopter la démarche suivante pour la conduite des opérations.

A partir d'un tube du lot de semences secondaire on ensemence des boites de Pétri qui, après cinq jours d'incubation, foumissent une suspension de spores.

Cette suspension de spores est utilisée pour ensemencer des fioles Erlenmeyer agitées contenant un milieu approprié. On peut aussi ensemencer directement la fiole agitée avec un tube du lot de semence. La culture en fioles agitées peut durer de deux à sept jours mais une durée de trois à cinq jours est préférée.

La production d'activité est observée dans le surnageant, dès le premier étage de culture en fiole mais il peut être avantageux de réaliser deux étages de culture successifs : un premier étage pour propager la biomasse, un second pour la production. Dans ce dernier cas une durée d'un ou deux jours est suffisante pour le premier étage.

L'activité antagoniste contenue dans les surnageants de culture en fioles est exprimée en $DI_{50}$ (dilution inhibitrice de 50 %, à savoir la dilution qui inhibe de 50 % la liaison du ligand à ses récepteurs) ; la $DI_{50}$ est généralement comprise entre 1/200 et 1/1000.

L'activité antagoniste des récepteurs du NPY est obtenue dans le surnageant des cultures en fioles mais il parait avantageux, pour obtenir une activité plus importante, de réaliser une culture en fermenteur et d'extraire ensuite le surnageant de celui-ci.

Le fermenteur est ensemencé par une culture en fiole agitée âgée d'un ou deux jours. En fermenteur, suivant le milieu de culture utilisé, l'activité antagoniste peut être observée dans le surnageant dès le premier jour mais il est avantageux de prolonger la culture au-delà de trois jours pour obtenir une production optimale.

Réaliser la culture de SEBR 2794 en fermenteur permet de mieux contrôler les conditions de culture qui sont décrites ci-après comme par exemple le pH ou l'aération.

L'activité antagoniste obtenue dans les surnageants de fermenteur, avant concentration, peut varier, selon les conditions de culture appliquées, entre une $DI_{50}$ de 1/500 et une $DI_{50}$ de 1/10000.

Le milieu de culture utilisé dans le procédé de fermentation doit contenir au moins une source de carbone assimilable, une source d'azote assimilable et des éléments minéraux. Comme sources de carbone assimilable, on peut utiliser des hydrates de carbone, tels que le glucose, le mannose, le maltose, des dextrines, le glycérol, les acides aminés et les protéines. Comme source de carbone assimilable, on peut aussi utiliser les acides acétique, subérique, citrique, propionique, succinique et 2-cétoglutarique ou certaines huiles animales ou végétales.

Les meilleures sources d'azote assimilable se trouvent parmi les protéines, les peptones et les acides aminés. Ces sources comprennent, par exemple, la caséine, la lactalbumine, le gluten et leurs hydrolysats, la farine de poisson, les extraits de levure ou les peptones.

La production de biomasse peut être augmentée par l'adjonction, au cours de la culture, de l'un et/ou de l'autre de ces deux principaux substrats.

Parmi les éléments minéraux ajoutés au milieu de culture pour assurer la croissance du micro-organisme et optimiser l'assimilation des sources de carbone et d'azote par les cellules du micro-organisme, il y a des sels de potassium, sodium, fer, magnésium, calcium, manganèse ainsi que les composés du phosphore tels que les phosphates et les oligoéléments.

Pour cultiver la source SEBR 2794 sur un milieu contenant ces composants, le procédé de culture sous aération et agitation, dans lequel on utilise un milieu liquide, est avantageux, bien que la culture sur un milieu gélosé puisse également être utilisée.

La température, la durée d'incubation, l'aération et le pH du milieu doivent être tels qu'ils donnent lieu à une croissance maximale du micro-organisme utilisé et un rendement maximum en extraits à activité antagoniste des récepteurs du NPY; la culture agitée pendant environ 2 à 7 jours est habituellement avantageuse.

De préférence, on maintient le pH du milieu de culture à une valeur à peu près neutre ou très faiblement basique et la température optimum d'incubation se situe entre environ 23°C et 35°C, l'intervalle préféré étant 25°C à 33°C.

Les conditions de culture telles que la composition et le pH du milieu, la température d'incubation, la vitesse d'agitation ainsi que l'aération de la fermentation peuvent varier dans de larges limites et devraient évidemment être choisies de façon à obtenir les meilleurs résultats possibles.

Pour obtenir les extraits antagonistes des récepteurs du NPY produits au cours de la culture, on sépare le surnageant du mycélium après avoir congelé ou non le moût de fermentation. Pour cette séparation on peut utiliser la centrifugation, la filtration par filtre presse, la filtration clarifiante, c'est-à-dire une filtration en présence d'un adjuvant de filtration ou toute autre technique habituellement utilisée pour extraire un produit extracellulaire d'un moût de fermentation.

L'extrait actif à usage cosmétique est préparé à partir du surnageant de culture obtenu.

Le surnageant peut être concentré par une technique membranaire ou par toute autre méthode de concentration afin de faciliter le conditionnement ou l'utilisation de la solution active.

On peut ainsi obtenir des activités avec des $DI_{50}$ variant de 1/250 à 1/50000.

Le surnageant, qu'il ait été concentré ou non, peut être dilué dans différents solvants compatibles avec une utilisation cosmétologique.

L'extrait ainsi obtenu selon cet aspect particulier de la présente invention est filtré sur 0,2 µm afin d'éliminer toute trace de biomasse résiduelle et d'assurer la propreté microbiologique : on le conditionne ensuite aseptiquement dans des flacons stériles et on obtient la solution active utilisable en cosmétologie.

Des fluides contenant les extraits NPY-antagonistes de la présente invention dans un glycol, de préférence dans le propylèneglycol constituent un autre aspect, très avantageux de la présente invention. Si l'on désire obtenir une poudre au lieu d'une solution active on peut simplement lyophiliser le filtrat.

Un dosage d'activité antagoniste aux récepteurs du NPY réalisé sur une partie aliquote permet d'évaluer l'activité de la solution active et de vérifier la reproductibilité du procédé.

Au niveau du surnageant ou du lyophilisat, on peut purifier l'extrait de l'invention de façon plus ou moins poussée selon les techniques conventionnelles de purification des "biomolécules", des polymères, des substances protéiques ou autres, telles que, par exemple, la chromatographie de perméation de gel, l'ultrafiltration, la chromatographie par adsorption, la chromatographie à contre-courant ou encore l'électrofocalisation.

Les extraits issus de cette nouvelle souche ont une activité antagoniste des récepteurs du NPY très intéressante. Leur puissante affinité pour ces récepteurs, tant pour les soustypes Y1 qu'Y2, a été démontrée au niveau de l'adipocyte de chien, ce dernier modèle présentant un haute homologie avec d'adipocyte humain.

Plus particulièrement, les membranes d'adipocytes ont été obtenues à partir du tissu omental adipeux prélevé chez le chien en utilisant essentiellement la technique décrite par Taouis et al., J. Pharmacol. Exp. Ther (1987), 242, 1041-1049. Les études de liaison ont été réalisées selon les techniques conventionnelles connues.

En particulier, on incube les membranes d'adipocytes (200 µg/ml) pendant 60 minutes à 30°C dans un milieu tamponné de liaison (solution de Krebs-Ringer Hepes 20 mM, pH=7,4, 1% sérum albumine bovine, 0,25 mg/ml bacitracine) avec 0,08 nM de [$^{125}$I]-NPY marqué par le réactif de Bolton-Hunter (Amersham IM 170,2000 Ci/mmole) en présence ou en absence de 0,3µM NPY porcin. On arrête l'incubation par filtration en utilisant des filtres Whatan GF/C et on évalue la radioactivité retenue par le filtre au compteur gamma.

La liaison non spécifique, mesurée en présence de 0,3 µM de NPY non marqué, représente 25% de la fixation totale. La préparation membranaire d'adipocytes de chien est porteuse de récepteurs $Y_1$ et $Y_2$ que l'on peut discriminer sur la base de l'affinité du fragment NPY (13-36) sélectif pour les récepteurs $Y_2$.

Les extraits selon la présente invention ont démontré une bonne affinité pour les récepteurs du NPY de ce tissu, en déplaçant de façon dose dépendante l'[$^{125}$I]-NPY fixé aux membranes d'adipocytes avec des $CI_{50}$ (c'est à dire des concentrations inhibitrices de 50% de la liaison spécifique du [$^{125}$I]-NPY) qui dépendent du degré de purification des extraits, et qui, dans le cas des extraits bruts directement obtenus des surnageants de fermentation par lyophilisation, sont de l'ordre au moins de $10^{-2}$ mg/ml.

Le caractère antagoniste du NPY des extraits selon cet aspect particulier de la présente invention a été mis en évidence par des études sur organes isolés et plus particulièrement dans le modèle du *"vas deferens*" du rat. Les extraits ont montré des propriétés antagonistes des effets du NPY dans le modèles Y2 du *vas deferens* de rat et on été étudiés selon le protocole décrit dans Regulatory Peptides 1986, 13, 307-318.

Cette activité a été confirmée sur adipocytes humains isolés du tissu adipeux sous-cutané, en dosant la libération des acides gras dans le milieu de culture et en la comparant au témoin (incubation sans produit).

Les solutions à 50 % (p/p) de ces extraits dans le propylène glycol sont particulièrement préférées.

La préparation aseptique de ces solutions permet d'éviter l'ajout de conservateur.

Dans la préparation des compositions selon la présente invention, les extraits à activité antagoniste des récepteurs du NPY, sont mélangés aux solvants aqueux ou non et aux diluants conventionnels compatibles avec une utilisation topique ainsi qu'avec les composants actifs de la composition même. Solvants et /ou diluants appropriés seront choisis selon leur capacité à véhiculer le composant actif de l'extrait de l'invention dans la couche adipeuse sous-cutanée.

Ces compositions contiennent généralement des excipients ou des additifs choisis parmi les ingrédients habituellement utilisés dans des compositions destinées à une application locale selon la nécessité des formulations particulières envisagées.

Elles peuvent contenir par exemple, des agents épaississants, des adoucissants, des émollients, des stabilisants, des conservateurs, des agents anti-mousse, des tensioactifs, des antioxydants, des colorants et/ou des pigments, des parfums.

Elles peuvent également contenir d'autres composants actifs ayant soit un effet du même type, par exemple, des produits qui contribuent à la régulation de la lypolyse/lipogénèse ou des produits utiles dans ce type de composition tels que des stimulateurs de la synthèse de collagène, des inhibiteurs de la collagénase ou de l'élastase, des vasoprotecteurs.

Les compositions de la présente invention contiennent outre le NPY-antagoniste, qui consiste en le produit

de fermentation de la souche Streptomyces sp SEBR 2794, déposée auprès de la CNCM de l'Institut Pasteur sous le numéro I-1332, ou en un mutant producteur de celle ci, et un α2-antagoniste qui peut-être un composé de synthèse non peptidique; un peptide ou un produit obtenu par fermentation d'un micro-organisme, par exemple d'une bactérie ou d'un champignon ou par extraction de cellules ou de tissus d'origine végétale ou animale.

Des α2-antagonistes avantageux à utiliser comme composants additionnels, à côté du NPY-antagoniste sont ceux des classes A et B ci-dessous.

A. Des produits de synthèse choisis parmi ceux des groupes I à VIII ci-après.

I - Les composés décrits dans BE 840363, notamment la mirtazapine.

II - Les composés décrits dans DE 2 603 407, notamment la setiptiline et ses analogues structuraux.

III - Les composés décrits dans US 4,337,260, notamment la mosapramine,

IV - Les composés décrits dans US 2,979,511, notamment l'idazoxan.

V - Les composés décrits dans WO 92/13856, notamment des 5-thiazolyl-N,N-diméthyltryptamines dont le CP 93393.

VI - Les composés décrits dans US 4,229,449, notamment la reboxetine.

VII - Les composés décrits dans GB 2 157 631, notamment le fluparoxan.

VIII - Les composés décrits dans GB 2 167 408, notamment l'altipamézole.

B. Des produits α2-antagonistes obtenus par extraction de cellules ou de tissus d'origine animale ou végétale ou par fermentation de micro-organismes, notamment de bactéries et de champignons, par exemples de levures. Des exemples de produits de cette classe, incluant également les produits d'hémisynthèse, sont les produits des groupes IX et X ci-après.

IX - Les extraits de l'ergot, leurs composants et composés d'hémisynthèse, ayant une action α2-antagoniste, notamment la nicegoline.

X - Les produits, notamment les extraits issus de la fermentation de souches de *Bacillus licheniformis* à activité antagoniste du récepteur α2, notamment de la souche SEBR 2464.

Ces produits, notamment les extraits appartenant à ce groupe, représentent un autre aspect particulier de la présente invention.

L'organisme producteur selon cet aspect particulier de la présente invention est une souche de *Bacillus licheniformis* qui a été isolée, auquel on a attribué le nom de code SEBR 2464. Un échantillon de ce microorganisme a été déposé le 22 octobre 1996 auprès de la C.N.C.M. de l'Institut Pasteur où il a été enregistré sous la référence I-1778.

Les caractéristiques de ce microorganisme ont été déterminées sur galeries A.P.I. API 20B, API 50 CHB, 50 CH, 50 AA, 50AO et 20E.

Il s'agit d'une bactérie en forme de bâtonnets mobiles de formes régulières, de 2 à 10 µm de longueur et de 0,5 à 1 µm de largeur, isolés ou en courtes chaînettes.

Comme la plupart des Bacillus cette bactérie est gram+, anaérobie facultative, catalase positive, oxydase négative et présente la caractéristique de sporuler sous certaines conditions.

Elle pousse bien à pH 5 à 7, à des températures comprises entre 15 et 55°C et pour des salinités (NaCl) jusqu'à 7 %.

Cette souche a été isolée comme contaminant, au cours d'expérimentations utilisant des colonnes de sable, selon les techniques microbiologiques classiques connues de l'homme de l'art.

Après cultures sur milieu nutritif gélosé et plusieurs repiquages successifs qui permettent d'obtenir une culture abondante et pure de la souche d'intérêt, on fabrique un lot 0 de conservation de la souche mère puis des lots de semence primaire et secondaire.

Pour cela, on prépare une suspension de spores à partir d'une culture sur milieu nutritif gélosé en boîte de Pétri et d'un milieu de reprise ; ce milieu contient un cryoprotectant permettant d'assurer une bonne viabilité des spores lors de la conservation par congélation.

La suspension de spores obtenue est répartie dans des cryotubes qui seront conservés à -80°C : ces tubes constituent le lot 0.

En suivant le même protocole, mais à partir d'un tube du lot 0, on prépare un lot de semence primaire.

Puis toujours selon le même protocole on prépare un lot de semence secondaire à partir d'un cryotube du lot de semence primaire.

La fabrication des lots de semences 0,1 et 2 assure une accessibilité durable de la souche et donc de l'activité recherchée.

Le procédé de préparation des extraits à activité antagoniste au récepteur $\alpha_2$ consiste essentiellement à cultiver la nouvelle souche SEBR 2464, ou ses mutants producteurs, sur un milieu et dans des conditions de cultures appropriées et à extraire ensuite du surnageant de culture la fraction active.

Ce surnageant peut être concentré jusqu'à l'obtention d'un extrait sec.

La culture de *Bacillus licheniformis* SEBR 2464 peut être effectuée par toute méthode de culture aérobie et dans différents types d'appareils habituellement utilisés dans l'industrie des fermentations. On peut, en particulier, adopter la démarche suivante pour la conduite des opérations.

A partir d'un tube du lot de semence secondaire, on ensemence des boîtes de Pétri, qui après deux jours d'incubation permettent d'inoculer des fioles Erlenmeyer agitées contenant un milieu approprié.

On peut aussi ensemencer directement une fiole agitée avec un tube du lot de semence. Dans ce cas, pour un même milieu, la durée de culture sera supérieure.

L'activité antagoniste est obtenue dans les cultures en fioles dans un délai de 10 heures à 48 heures suivant les conditions de cultures utilisées.

L'activité antagoniste au récepteur $\alpha_2$ est extraite des surnageants de culture.

L'activité des extraits est exprimée en $DI_{50}$ (dilution inhibitrice de 50 %) ; un litre de culture permet d'obtenir 10 ml d'extrait ayant une $DI_{50}$ comprise entre 1/2500 et 1/10 000.

L'activité antagoniste du récepteur $\alpha_2$ peut être obtenue par extraction du surnageant des cultures en fioles mais il paraît avantageux, afin d'obtenir une activité plus importante, de réaliser une culture en fermenteur et d'extraire ensuite le surnageant de celui-ci.

Le fermenteur est ensemencé par une culture en fiole agitée âgée de 1 à 2 jours, il est préférable que la culture n'ait pas commencé à sporuler.

En fermenteur, suivant les conditions de culture utilisées, l'activité antagoniste peut être observée dès les premières heures de la culture mais il est avantageux d'attendre que la phase de croissance stationnaire soit atteinte avant d'extraire.

La réalisation de la culture de SEBR 2464 en fermenteur permet de mieux contrôler les conditions de culture qui sont décrites ci-après par exemple le pH et l'aération.

L'activité antagoniste du récepteur $\alpha_2$ obtenue en fermenteur peut varier, selon les conditions de culture appliquées.

Un litre de culture permet d'obtenir à partir du surnageant, après extraction, 10 ml d'extrait présentant une $DI_{50}$ comprise entre 1/3 000 et 1/15 000.

Les caractéristiques du milieu sont identiques à celles décrites précédemment pour la souche SEBR 2794.

Afin de cultiver la souche SEBR 2464 sur un milieu contenant ces composants, le procédé de culture sous aération et agitation, dans lequel on utilise un milieu liquide est avantageux, bien que la culture sur un milieu gélosé puisse également être utilisée.

La température, la durée d'incubation, l'aération et le pH du milieu doivent être tels qu'ils donnent lieu à une croissance maximale du microorganisme utilisé et un rendement maximum en extrait à activité antagoniste du récepteur $\alpha_2$.

La culture agitée et aérée pendant environ 10 heures à 48 heures est habituellement avantageuse.

De préférence, on maintient le pH du milieu de culture à une valeur à peu près neutre ou très faiblement acide et la température optimum d'incubation se situe entre 25°C et 50°C.

Les conditions de culture telles que la composition et le pH du milieu, la température d'incubation, la vitesse d'agitation ainsi que l'aération de la fermentation peuvent varier dans de larges limites et devraient évidemment être choisies de façon à obtenir les meilleurs résultats possibles.

L'obtention de l'extrait présentant une forte activité antagoniste du récepteur $\alpha_2$ demande plusieurs étapes d'extraction.

Une première étape consiste à éliminer la biomasse. Pour cela on peut utiliser la centrifugation, la microfiltration tangentielle, la filtration clarifiante c'est-à-dire une filtration en présence d'un adjuvant de filtration ou toute autre méthode habituellement utilisée pour extraire un produit extracellulaire d'un moût de fermentation. Le surnageant est ensuite mis en contact pendant une nuit avec une résine hydrophobe de préférence en polystyrène divinylbenzène telle que par exemple la résine Amberlite $XAD_2$ (Rohm Hass) ou la résine CHP20P (Mitsubishi)

La résine chargée est alors séparée par filtration frontale, le filtrat est éliminé.

La résine subit plusieurs extractions successives par différents solvants permettant d'extraire les molécules à caractère hydrophobe. Après chaque extraction la résine est séparée de la phase organique par filtration.

Les différentes phases organiques sont ensuite évaporées sous vide, ensemble ou séparément, jusqu'à l'obtention d'un ou de plusieurs extraits secs.

Un dosage d'activité antagoniste du récepteur $\alpha_2$ réalisé sur une partie aliquote de chacun des extraits permet d'évaluer leur activité et de vérifier la reproductibilité du procédé.

L'extrait sec ainsi obtenu peut être repris dans différents solvants habituellement utilisés en cosmétologie.

La concentration de reprise est choisie pour permettre une parfaite dissolution de l'extrait et pour être compatible avec l'utilisation ultérieure. Afin d'éliminer toute trace de biomasse résiduelle et assurer sa stabilité microbiologique, l'extrait est filtré sur 0,2 µm et réparti aseptiquement dans des flacons stériles.

L'activité $\alpha_2$-antagoniste des extraits a été évaluée en utilisant la technique décrite par Chapleo CB et al., J. Med. Chem., 1983, 26, 823-831, sur le déplacement *in vitro,* au niveau du cortex de rat, du ligand de référence antagoniste des récepteurs $\alpha_2$ : l'idazoxan tritié.

L'extrait $\alpha_2$-antagoniste, à raison de 30 % d'extrait-sec, est introduit dans un mélange propylèneglycol-eau, 50-50 et constitue une dilution préférée selon l'invention.

On peut également utiliser des dilutions de préparation aseptique de ces solutions ce qui permet d'éviter l'ajout de conservateur.

Dans la préparation des compositions selon la présente invention, les extraits ainsi constitués à activité antagoniste des récepteurs $\alpha_2$, sont mélangés aux solvants aqueux ou non et aux diluants conventionnels compatibles avec une utilisation topique ainsi qu'avec les composants actifs de la composition même. Solvants et /ou diluants appropriés seront choisis selon leur capacité à véhiculer le composant actif de l'extrait de l'invention dans la couche adipeuse sous-cutanée.

Ces compositions contiennent généralement des excipients ou des additifs choisis parmi les ingrédients habituellement utilisés dans des compositions destinées à une application locale selon la nécessité des formulations particulières envisagées.

Elles peuvent contenir par exemple, des agents épaississants, des adoucissants, des émollients, des stabilisants, des conservateurs, des agents anti-mousse, des tensioactifs, des antioxydants, des colorants et/ou des pigments, des parfums.

Elles peuvent également contenir d'autres composant actifs ayant soit un effet du même type, par exemple, des produits qui contribuent à la régulation de la lypolyse/lipogénèse ou des produits utiles dans ce type de composition typique tels que des stimulateurs de la synthèse de collagène, des inhibiteurs de la collagénase ou de l'élastase, des vasoprotecteurs.

Les extraits $\alpha_2$-antagonistes de l'invention se sont, en plus, montrés complètement dépourvus de génotoxicité dans les tests de Ames et de la réparation de l'ADN. Leur stabilité est compatible avec leur utilisation dans des compositions cosmétiques.

Les compositions cosmétiques de la présente invention contiennent le NPY-antagoniste en pourcentages compris de 0,00001 % à 5 % par rapport au poids total de la composition, en mélange avec les excipients communément utilisés pour la préparation de formulations cosmétiques à appliquer sur la peau.

Lesdits pourcentages peuvent varier dans l'intervalle indiqué ci-dessus en fonction de l'acitivité intrinsèque du composant NPY-antagoniste indu dans la composition.

De préférence ledit composant NPY-antagoniste est présent en pourcentages de 0,0001 % à 2 %.

Avantageusement, le composant NPY-antagoniste est choisi parmi les produits inclus dans les classes A,B et C ci-dessus, ceux de la classe C étant particulièrement avantageux. Les composants appartenant aux groupes XV, XVI et XVII sont préférés.

Dans la préparation des compositions selon la présente invention, le composant NPY-antagoniste est mélangé aux solvants aqueux ou non et aux diluants conventionnels compatibles avec une utilisation sur la peau ainsi qu'avec d'autres composants de la composition même. Solvants et /ou diluants appropriés seront choisis selon leur capacité à déposer le composant NPY-antagoniste actif de l'invention sur la peau.

Ces compositions contiennent généralement des excipients ou des additifs choisis parmi les ingrédients habituellement utilisés dans des compositions destinées à une application locale selon la nécessité des formulations particulières envisagées.

Elles peuvent contenir par exemple, des agents épaississants, des adoucissants, des émollients, des stabilisants, des conservateurs, des agents anti-mousse, des tensioactifs, des antioxydants, des colorants et/ou des pigments, des parfums.

Lorsque le composant NPY-antagoniste est un extrait de cellules ou de tissus d'origine animale ou végétale ou un produit notamment un extrait, obtenu par fermentation d'un micro-organisme, par exemple d'une bactérie ou d'un champignon, la quantité de composant NPY-antagoniste est toujours de 0,00001 % à 5 %, de préférence de 0,0001 à 2 % par rapport au poids total de la composition, ladite quantité étant calculée par rapport au poids de l'extrait

sec.

Les compositions selon la présente invention comprennent un extrait obtenu de la fermentation de la nouvelle souche SEBR 2794 dans des proportions, en pourcentage poids/poids, qui dépendent du degré d'activité antagoniste des récepteurs du NPY de l'extrait utilisé et donc de la concentration en substance sèche et de l'activité spécifique de cette substance.

Plus l'activité antagoniste vis-à-vis des récepteurs du NPY de l'extrait utilisé est élevée, plus la quantité en poids, nécessaire pour obtenir l'effet lipolytique désiré est faible et *vice et versa.*

Pour obtenir les compositions cosmétiques selon l'invention, on peut convenablement utiliser les extraits bruts (surnageants filtrés sur 0,2 μm) obtenus directement de la fermentation sans aucune étape de purification, dans des proportions comprises de 0,00001 à 5%, avantageusement de 0,0001 à 2 % mieux de 0,01 à 2 % du poids et de préférence de 0,1 à 2% en poids. Ces pourcentages en poids sont naturellement calculés sur la base du poids d'extrait préparé.

Les compositions de la présente invention contiennent, en plus du composant NPY-antagoniste, aussi un composant α2-antagoniste, ce dernier est présent en pourcentage de 0,00001 à 5 %, avantageusement de 0,0001 à 2 %, mieux de 0,001 à 1 % de préférence à 0,01 à 0,5 %, par rapport au poids total de la composition.

Lesdits pourcentages peuvent varier dans les intervalles indiqués ci-dessus en fonction de l'activité intrinsèque du composant α2-antagoniste inclus dans la composition.

Avantageusement, le composant α2-antagoniste est choisi parmi les produits inclus dans les classes A et B ci-dessus, ceux de la classe B étant particulièrement avantageux. Les composés appartenant au groupe X sont préférés.

Lorsque le composant α2-antagoniste est un extrait de cellule ou de tissus d'origine animale ou végétale ou un produit obtenu par fermentation d'un micro-organisme, par exemple d'une bactérie ou d'un champignon, la quantité de composant α2-antagoniste est celle indiquée ci-dessus, ladite quantité étant calculée en poids de l'extrait sec.

Selon un aspect préférentiel, la composition de la présente invention contient un composant NPY-antagoniste susceptible d'être obtenu par fermentation de la souche *Streptomyces sp* SEBR 2794 déposée auprès de la CNCM de l'Institut Pasteur sous le numéro I 1332, ou de ses mutants producteurs et un composant α2-antagoniste susceptible d'être obtenu par fermentation de la souche *Bacillus licheniformis*.SEBR 2464 déposée auprès de la CNCM de l'Institut Pasteur sous le numéro I-1778, avec des excipients normalement utilisés pour des formulation de ce genre, tels que ceux mentionnés ci-dessus.

La forme des compositions selon la présente invention peut être une émulsion dans laquelle le constituant ou le mélange de constituants, avec un stabilisant éventuel, est associé aux excipients couramment utilisés dans les compositions cosmétiques et compatibles avec lesdits constituants tels que la lanoline ou les huiles végétales, minérales ou synthétiques.

Les compositions de l'invention peuvent être également présentées sous forme de gel dans des excipients appropriés tels que les esters de cellulose ou d'autres agents gélifiants comme les dérivés acryliques et contenir le principe actif sous forme dissoute ou suspendue en microgranuies.

Les compositions selon l'invention peuvent aussi prendre la forme d'une lotion ou d'une solution dans laquelle le mélange des constituants est dissout ou microdispersé.

La forme des compositions selon l'invention peut donc être une microdispersion dans un liquide contenant de l'eau ainsi qu'un ou plusieurs agents tensioactifs compatibles. Les dispersions présentent les caractères des microémulsions et ont pratiquement l'aspect de solutions vraies. Elles peuvent également être préparées extemporanément.

Une forme intéressante des compositions selon l'invention est un fluide appliqué topiquement par l'intermédiaire d'un support adhésif, ci-après désigné « patch », ce patch permettant une diffusion contrôlée des composants actifs éventuellement activée par des phénomènes physiques tels que microcourants électriques.

Les compositions cosmétiques de la présente invention peuvent également contenir d'autres composant actifs ayant soit un effet du même type que celui des NPY-antagonistes, par exemple, des produits qui contribuent à la régulation de la lypolyse/lipogénèse ou des stimulateurs de la synthèse de collagène, des inhibiteurs de la collagénase ou de l'élastase, des vasoprotecteurs.

Les compositions de la présente invention jouissent d'une bonne stabilité et peuvent être conservées pendant le temps nécessaire pour l'utilisation à des températures comprises entre 0°C et 60°C sans qu'il y ait sédimentation des constituants ou séparation des phases, ni une diminution de l'activité qui puisse en compromettre l'utilisation.

Ces compositions sont très bien tolérées, elles ne présentent aucune phototoxicité et leur application sur la peau, pour des périodes prolongées n'implique aucun effet secondaire.

Les compositions de la présente invention, dans leurs différentes formes de présentation, peuvent être utilisées outre comme régulateurs de la lipolyse/lipogénèse au niveau de la peau, comme produits cosmétiques à visée régulatrice de la séborrhée.

Les composition cosmétiques de la présente invention peuvent être mises en contact avec l'épiderme ou le

système pileux ou capillaire de façon à en modifier l'aspect et à les protéger.

Par exemple, lorsque ces compositions sont mises en contact avec l'épiderme, celui-ci prend un aspect « sain » comme si il avait été exposé à l'air et/ou au soleil, sans pour autant bronzer, si on ne le souhaite pas. Plus particulièrement, les compositions cosmétiques de la présente invention font perdre à la peau l'aspect « gras » avec le résultat d'amincir la partie du corps avec laquelle ladite composition est mise contact et de la maintenir en bon état. Dès les premières applications, le relief cutané est lissé, la peau devient plus tonique et plus ferme. Après un mois d'application, l'effet minceur apparait, l'aspect « peau d'orange » s'estompe visiblement et la silhouette s'affine.

Lorsque les compositions de la présente invention sont mises en contact avec le système pileux ou capillaire, par exemple après un traitement spécifique antiséborrhéique, ledit système est maintenu en bon état.

De même, les compositions de la présente invention maintiennent la peau'du visage en bon état, rendant aussi plus difficile la formation de comédons. Un fluide amincissant obtenu selon l'EXEMPLE 12 ci-dessous a été testé chez 50 volontaires de sexe féminin qui ont utilisé ledit fluide appliqué deux fois par jour sur les cuisses, avec un très bon résultat.

Le fluide amincissant décrit dans l'EXEMPLE 13 ci-dessous a été testé dans une étude incluant 150 volontaires de sexe féminin, effectuée en double aveugle contre placébo. L'analyse et la comparaison des résultats obtenus avec ce fluide amincissant e: avec son placébo dans les conditions expérimentales adoptées proches des conditions normales d'utilisation pour des applications répétées pendant 60 jours consécutifs sur les deux cuisses et l'abdomen de la taille au genou, ont permis de mettre en évidence un effet amincissant net et statistiquement significatif en faveur du fluide amincissant selon l'invention par rapport au placébo.

L'efficacité et la tolérance d'un fluide amincissant tel que décrit dans l'EXEMPLE 14 a été testé sur plus de 1000 femmes. Ces test ont montré une diminution de l'épaisseur du tissu adipeux, une action tonifiante et raffermissante et un amincissement, mis en évidence par mésure centimétrique , échographique et par absorptiométrie biphotonique.

L'invention a également pour objet une méthode de traitement cosmétique pour réaliser un effet amincissant caractérisée en ce que l'on applique sur des parties choisies du corps humain une quantité à effet cosmétique d'un NPY-antagoniste consistant en le produit de fermentation de la souche Streptomyces sp SEBR 2794, tel que défini ci-dessus et une quantité à effet cosmétique d'un $\alpha_2$-antagoniste, dans un véhicule à usage cosmétique. Les parties choisies du corps humain sont les cuisses et l'abdomen depuis la taille jusqu'aux genoux.

Un autre objet de la présente invention est une méthode de traitement cosmétique destinée à atténuer l'apparence grasse, de "peau d'orange" de la peau, comprenant l'application sur la peau d'une composition comprenant un composant NPY-antagoniste consistant en le produit de fermentation de la souche Streptomyces sp SEBR 2794, déposée auprès de la CNCM de l'Institut Pasteur sous le numéro I-1332, ou en un mutant producteur de celle ci, et un composant $\alpha$2-antagoniste en combinaison avec un excipient cosmétiquement acceptable.

Les exemples suivants illustrent l'invention sans cependant la limiter.

## EXEMPLE 1

Préparation d'un extrait issu de la souche *Streptomyces sp* SEBR 2794 (cf. XVII ci-dessus)

### 1.1 Fermentation

La fermentation de la souche *Streptomyces sp* SEBR 2794 a été conduite dans six milieux de culture différents.

1.1.1.

(a) On propage la souche en boîte de Pétri sur un milieu de repiquage :

| | |
|---|---|
| Glucose | 20 g |
| Soyoptim (SiO) | 10 g |
| $CaCO_3$ (OMYA) | 3 g |
| Agar type E | 20 g |
| Eau distillée q.s.p. | 1l |

On fait alors incuber la culture pendant 5 jours à 28°C. On obtient ensuite une suspension de spores en ajoutant dans chaque boîte de Pétri 15 ml d'un mélange approprié ayant la composition suivante :

| | |
|---|---|
| NaCl | 9,00 g |
| KCl | 0,42 g |
| $CaCl_2$ | 0,48 g |
| $NaHCO_3$ | 0,20 g |
| Glycérol | 150,00 g |
| Acide 3-[N-morpholino]propanesulfonique (MOPS) | 3,00 g |
| Eau distillée q.s.p. | 1 l |

(b) On utilise 3 ml de cette suspension pour ensemencer 100 ml de milieu de culture ayant la composition suivante :

| | |
|---|---|
| Glucose | 30 g |
| Soyoptim (SIO) | 15 g |
| Tryptone USP (Biokar) | 2 g |
| Extrait de levure (Difco) | 5 g |
| Solution d'oligoéléments | 10 ml |
| $CaCO_3$ | 5,00 g |
| Eau distillée q.s.p. | 1 l |
| pH = 7 | |

où la solution d'oligoéléments est constituée par les composés suivants :

| | |
|---|---|
| $FeSO_4$,7 $H_2O$ | 1,00 g |
| $MnSO_4$, 4 $H_2O$ 1,00 | 1,00 g |
| $CuCl_2$, 2 $H_2O$ | 0,025 g |
| $CaCl_2$, 2 $H_2O$ | 0,10 g |
| $H_3BO_3$ | 0,56 g |
| $(NH_4)_6Mo_7O_{24}$, $4H_2O$ | 0,002 g |
| $ZnSO_4$, 7 $H_2O$ | 0,20 g |
| Eau q.s.p. | 1 l |

La culture est développée à 28°C pendant 72 heures dans les fioles Erlenmeyer de 500 ml en agitant à 220 tours/minute. Le pH est, en fin d'opération, de 7,5.

1.1.2

(a) On réalise une suspension de spores de la souche SEBR 2794 selon la méthode indiquée dans 1.1.1 (a) et
(b) On utilise 3 ml de cette suspension pour ensemencer 100 ml de culture ayant la composition suivante :

| | |
|---|---|
| Glucose | 10 g |
| Amidon soluble (Merck) | 30 g |
| Extrait de malt (Difco) | 5 g |
| Soyoptim (SIO) | 15 g |
| Trypton USP (Biokar) | 2 g |

(suite)

| | |
|---|---|
| Extrait de levure(Difco) | 5 g |
| Solution d'oligo-éléments (ayant la même composition que dans 1.1.1 (b)) | 10 ml |
| $CaCO_3$ | 5 g |
| Eau distillée q.s.p. | 1 l |
| pH = 7 | |

La culture est développée comme indiqué dans 1.1.1

1.1.3 On répète la procédure décrite dans les EXEMPLES 1.1.1 et 1.1.2 en changeant la composition du milieu de culture de l'étape (b) qui est la suivante :

| | |
|---|---|
| Glycérol (Prolabo) | 10 g |
| Amidon soluble (Merck) | 30 g |
| Soyoptim (SIO) | 15 g |
| Trypton USP (Biokar) | 2 g |
| Extrait de levure(Difco) | 5 g |
| Solution d'oligo-éléments (ayant la même composition que dans l'EXEMPLE 1) | 10 ml |
| $CaCO_3$ | 5 g |
| Eau distillée q.s.p. | 1 l |
| pH = 7 | |

La culture est développée comme indiquée dans 1.1.1.

1.1.4

(a) On utilise 0,5 ml de suspension de spores congelées contenus dans un cryotube du lot de semence pour inoculer une fiole de 500 ml contenant 100 ml de milieu de culture ayant la composition suivante :

| | |
|---|---|
| Extrait de levure (Difco) | 3 g |
| Extrait de malt (Difco) | 3 g |
| Peptone (Difco) | 5 g |
| Glucose | 10 g |
| Eau distillée q.s.p. | 1 l |

La culture est développée pendant 72 heures à 28°C en agitant à 220 tours/mn. Le pH du milieu en fin d 'opération est proche de 7,9.

1.1.5

(a) On procède selon la méthode indiquée dans la partie (a) de 1.1.4 mais on prépare trois cultures identiques que l'on arrête à 24 heures d'âge.

(b) Les trois cultures sont réunies et sont utilisées pour inoculer un fermenteur de 20 l contenant 10 l ce milieu ayant la composition suivante

| | |
|---|---|
| Extrait de levure | 3 g |
| Extrait de malt | 3 g |

(suite)

| | |
|---|---|
| Peptone | 5 g |
| Glucose * | 30 g |
| Eau distillée q.s.p. | 1 l |

(*) : Glucose stérilisé à part

La culture est développée pendant au moins 72 heures à 28°C. L'aération est fixée à 1 WM (volume d'air / volume de culture . minute) et la vitesse d'agitation est régulée de façon à maintenir une pression en oxygène dissous proche de 20 %. Le pH n'est pas régulé et évolue librement entre 7,2 et 6,3.

1.1.6

(a) On procède de la même façon que dans l'EXEMPLE 1.1.5 mais en utilisant un milieu de préculture (fioles) ayant la composition suivante :

| | |
|---|---|
| Extrait de levure (Difco) | 15 g/l |
| Glucose | 10 g/l |
| Eau distillée q.s.p. | 1 l |

(b) On procède de la même façon que dans l'EXEMPLE 1.1.5. mais en utilisant un milieu de culture (fermenteur) ayant la composition suivante :

| | |
|---|---|
| Extrait de levure (Difco) | 30 g/l |
| Glucose | 30 g/l |

| | |
|---|---|
| Antimousse (Struktol) | 1 ml |
| Eau distillée q.s.p. | 1 ml |

La culture est développée pendant 144 heures à 28°C.
L'aération est fixée à 1 WM (un volume d'air / volume de culture . minute) et la vitesse d'agitation est régulée de façon à maintenir une pression en oxygène dissous proche de 20 %. A 72 heures d'âge on ajoute 10 g/l de glucose pour prolonger la culture. Le pH n'est pas régulé et évolue librement entre 6,5 et 8.

**1.2. Extraction**

1.2.1. On réalise l'extraction sur 1,5 litre de moût obtenu selon l'EXEMPLE 1.1.1.
On centrifuge à 13000 tours/minute (27500 g) pendant 20 minutes et on filtre le moût de fermentation en présence de 15 g d'adjuvant de filtration sur filtre presse. Le filtrat obtenu est directement lyophilisé et le résidu sec ainsi obtenu (16,3 g) est remis en solution dans 80 ml d'eau (pH final = 8,4).
La dilution inhibitrice 50 de cette fraction ($ID_{50}$) est de 1/4500.

1.2.2. On réalise l'extraction sur 10 l de moût de culture obtenus en procédant selon l'EXEMPLE 1.1.5 et préalablement congelés.
On centrifuge en pots de 500 ml à 8 000 RPM (11.000 g) pendant 20 mn. On obtient 9 de sumageant parfaitement limpide.
Un échantillon aliquote est concentré cinq fois par concentrateur Centripep.
La dilution inhibitrice 50 de cette fraction est égale à 1/10 000.

1.2.3. On réalise l'extraction sur 10 l de moût de culture préalablement congelés et obtenus dans les mêmes

conditions que dans l'EXEMPLE 1.1.5.
On réalise la séparation sur une centrifugeuse continue de type Sharples.
Les 9 l de surnageant obtenus sont filtrés sur 0,2 μm pour éliminer toute trace de biomasse résiduelle.
On obtient 8,5 l de filtrat. Un échantillon aliquote est concentré cinq fois par concentrateur Centripep. La dilution inhibitrice 50 de cette fraction est de 1/16 000.

**1.3. Concentration par ultrafiltration**

1.3.1. Concentration sur membrane 5 kD
On procède avec une ultrafiltration de la solution de l'EXEMPLE 1.2.1 en utilisant une membrane Amicon 5000 en cellule à agitation en tampon phosphate 25 mM pH = 7,5 contenant NaCl 150 mM et on obtient un rétentat actif et un perméat totalement inactif.

1.3.2. Concentration sur membrane 10 kD.
Pour concentrer les 9 litres de surnageant obtenus dars l'EXEMPLE 2.2 on utilise une membrane Amicon de 10 kD En concentrant d'un facteur 5 on obtient 1.8 l de concentrat cinq fois plus actif ($DI_{50}$ = 1/10 000) et un perméat totalement inactif.

1.3.3 Concentration sur membrane 30 kD
Pour concentrer les 8,5 l de filtrat obtenus dans l'EXEMPLE 2.3 on utilise une membrane Amicon de 30 kD. En concentrant d'un facteur 5 on obtient 1,7 l de concentrat jusque cinq fois plus actif ($DI_{50}$ = 1/10 000) et un perméat contenant une activité très faible (20 % d'inhibition au 1/250).

**1.4 Purification**

1.4.1. Chromatographie de perméation de gel.
On soumet des échantillons de la solution de 1.2.1 à des chromatographies de perméation de gel en utilisant des gels différents : Sephadex G 25, G 50, G 75 et G 100 et on obtient les résultats indiqués ci-dessous :
L'activité est éluée au volume d'exclusion sur Sephadex G 25 et G 50 et très près du volume d'exclusion de G 75.
Sur gel Sephadex G 100, l'activité peut être séparée des produits de plus hauts poids moléculaire (> 100 KD).
Sur une colonne analytique de Superose 12 (domaine de fractionnement 1 KD à 100 KD), la molécule active a montré un temps de rétention compris entre celui de l'ovalbumine (44 KD) et celui du cytochrome (14,4 KD).

1.4.2. Ultrafiltration sur membrane de seuil de coupure 5000 Daltons.
On dialyse à 5 volumes de tampon phosphate 20 mM pH 7,4,NaCl 150 mM, 100 ml d'un surnageant de culture ($DI_{50}$ 1/3600) dans une cellule à agitation magnétique, équipée d'une membrane Amicon 5000, pressurisée à l'air comprimé à la pression d'$1.10^5$ Pa (1 bar).
On récupère 100 ml de retentat ($DI_{50}$ 1/2500) débarrasse des molécules de petits poids moléculaires et 500 ml d'ultrafiltrat d'activité négligeable.

1.4.3. Ultrafiltration sur membrane de seuil de coupure 10000 Daltons.
On dialyse à 7 volumes de tampon phosphate 20 mM pH 7,4,150 mM de NaCl, 100 ml d'un surnageant de culture ($DI_{50}$ 1/3600) dans une cellule à agitation magnétique. pressurisée à $1.10^5$ Pa (1 bar) d'air comprimé et équipée dune membrane Filtron 10K
On récupère 100 ml de rétentat ayant une activité $DI_{50}$ 1/1500 en inhibition du binding sur le recepteur NPY.
L'ultrafiltrat (700 ml) est reconcentré dans une cellule du même type équipée d'une membrane Amicon 1000.
On obtient 100 ml de solution présentant une $DI_{50}$ au 1/500.

1.4.4. Elimination des impuretés hydrophobes.
1500 ml d'un surnageant de culture sont traités pendant 2 heures, sous agitation avec 180 g de résine polystyrène divinylbenzène (XAD2). La résine est ensuite filtrée, lavée avec 2 fois 300 ml de méthanol, puis avec 300 ml d'acétone. Les solutions de méthanol et d'acétone rassemblées sont évaporées sous vide. Le résidu repris par 40 ml de méthanol fournit une solution ne présentant pas d'activité.
La solution aqueuse débarrassée des produits hydrophobes est lyophilisée.

Le résidu repris par 80 ml d'eau donne une solution ayant une $DI_{50}$ 1/4500 en inhibition du binding sur le récepteur NPY.

1.4.5. Extraction de la molécule active par échange de cations.
1500 ml d'un surnageant de culture sont ajustés à pH 5 avec de l'acide acétique N, puis traités avec 120 g de résine échangeuse de cations (DOWEX 50 WX-4, forme ACO).
Après une heure d'agitation, la résine est filtrée, puis désorbée par 300 ml d'ammoniaque 1N pendant une demi-heure. Cette solution est ensuite lyophilisée. Le résidu obtenu repris par 40 ml d'eau fournit une solution qui, mesurée en inhibition du binding sur le récepteur NPY, possède une $DI_{50}$ au 1/3600.

On peut également obtenir des solutions à activité antagoniste des récepteurs du NPY en réalisant l'extraction des moûts de culture des EXEMPLES 1.1.2 ; 1.1.3 ; 1.1.4 et 1.1.6 selon les EXEMPLES d'extraction décrits de 1.2.1 à 1.2.3.

EXEMPLE 2

Préparation d'un extrait issu de la souche *Bacillus licheniformis* SEBR 2464 (Cf. ci-dessus XXVII).

**2.1. Fermentation**

La fermentation de la souche *Bacillus licheniformis* SEBR 2464 a été conduite dans 4 milieux de culture différents

2.1.1

(a) On propage la souche en boites de Pétri sur un milieu de repiquage ayant la composition suivante.

| | |
|---|---|
| Bacto tryptic Soy Broth (Difco) | 40 g |
| MOPS | 2 g |
| Extrait de levure (Difco) | 5 g |
| Agar type E | 20 g |
| Eau distillée q.s.p. | 1 l |

On fait incuber la culture pendant 48 heures à 30°C. On obtient ensuite une suspension de spores en ajoutant dans chaque boite de Pétri 15 ml d'un milieu de reprise approprié ayant la composition suivante :

| | |
|---|---|
| NaCl | 9,00 g |
| KCl | 0,42 g |
| $CaCl_2$ | 0,48 g |
| $NaHCO_3$ | 0,20 g |
| Glycérol | 150,00 g |
| MOPS | 3,00 g |
| Eau distilliée q.s.p. | 1 l |

(b) On utilise 3 ml de cette suspension pour ensemencer 200 ml de milieu de culture ayant la composition suivante :

| | |
|---|---|
| Tryptone USP (Difco) | 30 g |
| Peptone papaïnique de soja (Difco) | 5 g |
| NaCl | 5 g |
| Eau distillée q.s.p. | 1 l |

(suite)

| | |
|---|---|
| pH = 7 | |

La culture est développée à 30°C pendant 24 heures dans des fioles Erlenmeyer de 500 ml en agitant à 260 tours/minute.

2.1.2 On utilise 1.5 ml de suspension de spores congelée du lot de semence secondaire pour inoculer une fiole de 500 ml contenant 100 ml de milieu de culture ayant la composition suivante :

| | |
|---|---|
| Glucose | 30 g* |
| Extrait de levure (Difco) | 10 g |
| $MgSO_4$, 7 $H_2O$ | 0,5 g |
| $KH_2PO_4$ | 1 g |
| Solution d'oligoéléments | 100 ml |
| Eau distillée q.s.p. | 1 l |

*Glucose stérilisé à part

où la solution d'oligoéléments est constituée par les composés suivants :

| | |
|---|---|
| $FeSO_4$, 7 $H_2O$ | 1,00 g |
| $MnSO_4$, 4 $H_2O$ | 1,00 g |
| $CuCl_2$, 2 $H_2O$ | 0,025 g |
| $CaCl_2$, 2 $H_2O$ | 0,10 g |
| $H_3BO_3$ | 0,56 g |
| $(NH_4)_6Mo_7O_{24}$, $4H_2O$ | 0,02 g |
| $ZnSO_4$, 7 $H_2O$ | 0,20 g |
| Eau distillée q.s.p. | 1 l |

La culture est développée à 30°C avec une agitation de 260 RPM.
La culture est arrêtée à 15 heures d'âge.

2.1.3. On prépare 300 ml de culture en fioles agitées de la même façon que dans l'EXEMPLE 2.1.2. et on utilise cette culture pour inoculer un fermenteur de 20 l contenant 10 de milieu de même composition auquel on ajoute 1 ml/l de strucktol. La culture est développée pendant 15 heures à 30°C avec une agitation de 260 RPM et une aération de 0,5 WM.

2.1.4.

(a) On prépare 5 cultures en fioles agitées de 1 l contenant 300 ml de milieu ayant la composition suivante :

| | |
|---|---|
| Glucose | 30 g |
| Extrait de levure (Difco) | 30 g |
| $MgSO_4$, $7H_2O$ | 0,5 g |
| $KH_2PO_4$ | 1 g |
| Solution d'oligoéléments | 10 ml |
| Eau distillée q.s.p. | 1 l |

La solution d'oligoéléments a la même composition que dans l'EXEMPLE 2.1.2.

(b) Les 5 cultures sont développées pendant 15 heures à 30°C et 220 RPM puis sont réunies pour inoculer un fermenteur de 75 l contenant 50 l de milieu ayant la composition suivante :

| | |
|---|---|
| Glucose | 30 g |
| Extrait de levure (Difco) | 30 g |
| $MgSO_4$, 7 $H_2O$ | 1 g |
| $KH_2PO_4$ | 2 g |
| Solution d'oligoéléments | 20 ml |
| Strucktol | 2 ml |
| Eau distilliée q.s.p. | 1 l |

La solution d'oligoéléments a la même composition que dans l'EXEMPLE 1.1.1.
La culture est développée à 30°C avec une aération de 0,5 WM et une agitation régulée de façon à maintenir une pression en oxygène dissous proche de 20 %. A 7 heures d'âge une solution de glucose concentrée est ajoutée pour prolonger la croissance. La culture est arrêtée au palier de croissance à 16 heures d'âge.

**2.2. Extraction**

2.2.1. On réalise l'extraction sur 12 l de moût de culture de l'EXEMPLE 2.1.1.
On centrifuge à 13000 tours/minute (27500 g) pendant 10 mn et on obtient 11 l de surnageant. A ces 11 l de surnageant est additionné 2.1 kg de résine Amberlite $XAD_2$ préalablement conditionnée par du méthanol et de l'acétone.
Le mélange surnageant/résine est mis en contact une nuit à température ambiante.
La résine est ensuite filtrée et les produits fixés sur celle-ci sont élués successivement par deux fois 5 litres de méthanol et par 5 litres d'acétone.
Les phases organiques sont réunies après filtration et évaporées à sec sous vide.
Le résidu d'évaporation (46 g) constitue l'extrait sec il est redissout dans 100 ml du mélange propylène glycol/eau (50/50).
La fraction ainsi obtenue a une $DI_{50}$ = 1/3000.

2.2.2. On réalise l'extraction sur 3,5 de moût de culture de l'EXEMPLE 2.1.3.
On suit le même protocole que dans l'EXEMPLE 2.2.1. mais en adaptant les quantités de résine et de solvant au volume de surnageant traité.
On obtient 11 g d'extrait sec qui sont repris avec 27 ml du mélange propylène glycol/eau (50/50).
La solution ainsi obtenue présente une $DI_{50}$ = 1/4000.

2.2.3. On réalise l'extraction sur 50 de moût de culture de l'EXEMPLE 2.1.4.
Pour séparer la biomasse on utilise un système de microfiltration tangentielle (0,2 µm). On obtient 46 l de perméat qui sont mis en contact avec 9 kg de résine Amberlite $XAD_2$ préconditionnée, pendant une nuit à température ambiante.
La résine chargée est ensuite filtrée et les produits fixés sur celle-ci sont élues par deux fois 23 l de méthanol et une fois 23 l d'acétone. Entre chaque élution, la phase organique et la résine sont séparées par filtration.
Les phases organiques sont ensuite réunies et évaporées à sec sous vide.
On obtient 285 g d'extrait sec que l'on reprend avec 620 g du mélange propylène glycolleau (50/50).
La solution obtenue est filtrée sur 0,2 µm et présente une $DI_{50}$ = 1/8200.

On peut également obtenir une solution a activité antagoniste du recepteur $\alpha_2$ en réalisant l'extraction du moût de culture de l'EXEMPLE 2.1.2 selon l'EXEMPLE 2.2.3.

**EXEMPLE 3**

On prépare la composition suivante pour l'application sur la peau sous forme de gel amincissant

| | |
|---|---|
| Carbopol 940 | 0.20 g |
| Polyéthylène glycol | 3,00 g |
| Conservateurs | 0,75 g |
| Tween 20 | 0,50 g |
| Triéthanolamine | 0,25 g |
| Escine | 0,50 g |
| Caféine | 0,50 g |
| Extrait de Centella asiatica | 3,00 g |
| Extrait de Ginkgo biloba | 3,00 g |
| Carnitine | 4,00 g |
| Extrait de l'EXEMPLE 2.2.1 | 0,10 g |
| Extrait sec de l'EXEMPLE 1.2.1 | 0,01 g |
| Eau déminéralisée q.s.p | 100,00 g |

**EXEMPLE 4**

On prépare la composition suivante utilisable pour l'application sur la peau comme émulsion amincissante :

| | |
|---|---|
| Carbopol 934 | 0,300 g |
| Triéthanolamine | 0,520 g |
| Escine | 0,500 g |
| Alcool cétylique | 1,500 g |
| Esters gras fluides | 8,500 g |
| Palmitate de cétyle | 2,000 g |
| Phytostérol | 1,000 g |
| Alcool cétylique PoE | 0,700 g |
| Huile de silicone | 2,500 g |
| Polysorbate 60 | 1,900 g |
| Sorbitan Stéarate | 1,400 g |
| Propylène glycol | 4,000 g |
| Conservateurs | 0,700g |
| Extrait de rate | 1,000 g |
| Carnitine | 4,000 g |
| Extrait de l'EXEMPLE 2 2.1 | 0,0015 g |
| Extrait sec de l'EXEMPLE 1.3.1 | 0,0010 g |
| Eau déminéralisée q.s.p | 100,00 g |

**EXEMPLE 5**

On prépare la composition suivante utilisable pour l'application sur la peau sous forme de microémulsion amincissante :

| | |
|---|---|
| Caféine | 0,50 g |

(suite)

| | |
|---|---|
| Escine | 0,50 g |
| Triglycérides éthoxylés | 25,70 g |
| Extrait de rate | 1,00 g |
| Carnitine | 4,00 g |
| Alcool gras éthoxylés | 12,00 g |
| Triglycérides synthétiques | 7,00 g |
| Huile de silicone | 3,50 g |
| Ester gras | 3,50 g |
| Conservateurs | 0,75 g |
| Extrait sec de l'EXEMPLE 1.4.3 | 0,004 g |
| Extrait de l'EXEMPLE 2.2.1 | 0,15 g |
| Eau déminéralisée q.s.p | 100,00 g |

EXEMPLE 6

Patch amincissant

| | |
|---|---|
| Adhésif* | QSP 100 |
| Extrait de l'EXEMPLE 1.3.2 | 0,020 |
| Extrait de l'EXEMPLE 2.2 3 | 0,001 |

* L'adhésif pouvant être du polyisobuténe un adhésif acrylique ou silicone ou tout autre adhesif biocompatible

**EXEMPLE 7**

Macroémulsion amincissante

| | |
|---|---|
| Glycérine | 5,000 |
| Huile minérale | 2,00 |
| Panthenol | 0,500 |
| Acrylates copolymer | 0,050 |
| Triéthanolamine | 0,050 |
| Parabens | 0,300 |
| Phénoxyéthanol | 0,700 |
| Extrait de l'EXEMPLE 1.3.2 | 0.020 |
| Extrait de l'EXEMPLE 2.2.3 | 0.001 |
| Eau deminéralisée q s p | 100.00 g |

**Revendications**

1. Composition pour le traitement de la cellulite par application topique, comprenant un composant NPY (neuropeptide Y)-antagoniste consistant en un produit de fermentation de la souche *Streptomyces sp* SEBR 2794, déposée auprès de la CNCM de l'Institut Pasteur sous le numéro I-1332, ou d'un mutant producteur de celle-ci et un composant α2-antagoniste en combinaison avec un excipient cosmétiquement acceptable.

**2.** Composition selon la revendication 1, comprenant de 0,00001 % à 5 % en poids du composant NPY-antagoniste et de 0,00001 % à 5 % en poids d'un composant α2-antagoniste.

**3.** Composition selon la revendication 1 ou 2, dans laquelle le composant α2-antagoniste est un produit de fermentation de la souche *Bacillus licheniformis* SEBR 2464, déposée auprès de la CNCM de l'Institut Pasteur sous le numéro I-1778, ou d'un mutant producteur de celle-ci.

**4.** Composition selon la revendication 1, contenant en outre un stimulateur de la synthèse du collagène, un inhibiteur de la collagénase ou de l'élastase ou un vasoprotecteur.

**5.** Méthode de traitement cosmétique pour réaliser un effet amincissant, comprenant l'application topique d'une composition selon la revendication 1, 2 ou 3, sur des parties choisies du corps humain.

**6.** Méthode selon la revendication 5, dans laquelle la composition de la revendication 3 est appliquée sur les cuisses.

**7.** Méthode selon la revendication 6, dans laquelle la composition de la revendication 3 est appliquée sur les cuisses et l'abdomen depuis la taille jusqu'aux genoux.

**8.** Méthode de traitement cosmétique destinée à atténuer l'apparence grasse, de "peau d'orange" de la peau, comprenant l'application sur la peau d'une composition selon l'une quelconque des revendications 1, 2 ou 3.

**Patentansprüche**

**1.** Zubereitung zur Behandlung der Cellulitis durch topische Anwendung, umfassend einen NPY (Neuropeptid Y)-antagonistischen Bestandteil, der aus einem Fermentationsprodukt des Stammes *Streptomyces sp.* SEBR 2794, der bei dem DNCM des Institut Pasteuer unter der Nummer 1-1332 hinterlegt worden ist, oder einer dieses bildenden Mutante besteht, und einen α2-antagonistischen Bestandteil in Kombination mit einem kosmetisch annehmbaren Trägermaterial.

**2.** Zubereitung nach Anspruch 1, umfassend 0,00001 % bis 5 Gew.-% des NPY-antagonistischen Bestandteils und 0,00001 % bis 5 Gew.-% eines α2-antagonistlschen Bestandteils.

**3.** Zubereitung nach Anspruch 1 oder 2, worin der α2-antagonistische Bestandteil ein Fermentationsprodukt ist des Stammes *Bacillus lichentformis* SEBR 2464, der bei dem CNCM des Institut Pasteur unter der Nummer I-1778 hinterlegt worden ist, oder einer dieses liefernden Mutante.

**4.** Zubereitung nach Anspruch 1, enthaltend zusätzlich einen Stimulator der Collagensynthese, einen Inhibitor der Collagenase oder der Elastase oder ein gefäßschützendes Mittel.

**5.** Verfahren zur kosmetischen Behandlung zur Erzielung eines Verschlankungseffekts, umfassend den topischen Auftrag einer Zubereitung nach Anspruch 1, 2 oder 3 auf die ausgewählten Bereiche des menschlichen Körpers.

**6.** Verfahren nach Anspruch 5, worin die Zubereitung nach Anspruch 3 auf die Schenkel aufgetragen wird.

**7.** Verfahren nach Anspruch 6, worin die Zubereitung nach Anspruch 3 auf die Schenkel und den Bauch von der Taille bis zu den Knien aufgetragen wird.

**8.** Verfahren zur kosmetischen Behandlung zur Verringerung des feisten Aussehens, der "Orangenhaut" der Haut, umfassend das Auftragen einer Zubereitung nach einem der Ansprüche 1, 2 oder 3 auf die Haut.

## Claims

1. Composition for the treatment of cellulite by topical application, comprising an NPY- (neuropeptide Y) antagonist component consisting of a product of fermentation of the strain *Streptomyces sp* SEBR 2794, deposited with the CNCM of the Institut Pasteur under the number I-1332, or of a producer mutant thereof, and an α2-antagonist component in combination with a cosmetically acceptable excipient.

2. Composition according to Claim 1, comprising from 0.00001% to 5% by weight of the NPY-antagonist component and from 0.00001% to 5% by weight of an α2-antagonist component.

3. Composition according to Claim 1 or 2, in which the α2-antagonist component is a product of fermentation of the strain *Bacillus licheniformis* SEBR 2464, deposited with the CNCM of the Institut Pasteur under the number I-1778, or of a producer mutant thereof.

4. Composition according to Claim 1, also containing a collagen synthesis stimulator, a collagenase or elastase inhibitor or a vasoprotector.

5. Method of cosmetic treatment for producing a slimming effect, comprising the topical application of a composition according to Claim 1, 2 or 3, to selected parts of the human body.

6. Method according to Claim 5, in which the composition of Claim 3 is applied to the thighs.

7. Method according to Claim 6, in which the composition of Claim 3 is applied to the thighs and abdomen from the waist to the knees.

8. Method of cosmetic treatment intended to lessen the fatty, "orange peel" appearance of the skin, comprising the application to the skin of a composition according to any one of Claims 1, 2 or 3.